# FASCICULE DE BREVET EUROPEEN

(11) **EP 3 294 899 B1**
(45) Date de publication et mention de la délivrance du brevet: **15.10.2025**
(21) Numéro de dépôt: 16727767.2
(22) Date de dépôt: 13.05.2016
(51) Int. Cl.: C12P 21/02, C12N 15/82

(54) **PROCEDE DE PRODUCTION DE PROTEINES A PARTIR D'UNE STRUCTURE VEGETALE**
VERFAHREN ZUR HERSTELLUNG VON PROTEINEN AUS PFLANZLICHEN STRUKTUREN
METHOD FOR PRODUCING PROTEINS FROM PLANT STRUCTURES

(30) Priorité: 15.05.2015 FR 1501002
(43) Date de publication de la demande: 21.03.2018
(73) Titulaire: Université de Picardie Jules Verne, 80025 Amiens Cedex 1 (FR)
(72) Inventeur: GUERINEAU, François, 80480 Salouel (FR); BOITEL-CONTI, Michèle, Aimée, Yvonne, 80000 Amiens (FR); ELE EKOUNA, Jean Pierre, 80080 Amiens (FR)
(74) Mandataire: Grosset-Fournier, Chantal Catherine
(86) Numéro de dépôt international: PCT/FR2016/051149
(87) Numéro de publication internationale: WO 2016/185122

(56) Documents cités:
- EP-A1- 2 385 130
- TRUEN N. QUACH ET AL: "Functional Analysis of Water Stress-Responsive Soybean GmNAC003 and GmNAC004 Transcription Factors in Lateral Root Development in Arabidopsis", PLOS ONE, vol. 9, no. 1, January 2014 (2014-01-01), pages 1 - 12
- BENJAMIN PÉRET ET AL: "Auxin regulates aquaporin function to facilitate lateral root emergence", NATURE CELL BIOLOGY, vol. 14, no. 10, 16 September 2012 (2012-09-16), GB, pages 991 - 998, XP055616380, ISSN: 1465-7392, DOI: 10.1038/ncb2573
- WASHIDA D ET AL: "Auxins affected ginsenoside production and growth of hairy roots in Panax hybrid", MEDICINAL & AROMATIC PLANTS ABSTRACTS, SCIENTIFIC PUBLISHERS, NEW DELHI - INDIA, vol. 26, no. 6, 1 December 2004 (2004-12-01), XP018002408
- I BÁLVÁNYOS ET AL: "The effect of plant growth regulators on biomass formation and lobeline production of Lobelia inflata L. hairy root cultures", PLANT GROWTH REGULATION, 1 July 2001 (2001-07-01), Dordrecht, pages 339 - 345, XP055254167, Retrieved from the Internet <URL:http://rd.springer.com/content/pdf/10.1023/A:1013374524757.pdf> [retrieved on 20160301], DOI: 10.1023/A:1013374524757
- AGOSTINI ELIZABETH ET AL: "Production of peroxidases by hairy roots of Brassica napus", PLANT CELL, TISSUE AND ORGAN CULTURE, SPRINGER, NL, vol. 47, no. 2, 1 January 1996 (1996-01-01), pages 177 - 182, XP002598288, ISSN: 0167-6857, DOI: 10.1007/BF02318955
- ZANG YUN-XIANG ET AL: "Metabolic Engineering of Indole Glucosinolates in Chinese Cabbage Hairy Roots Expressing Arabidopsis CYP79B2, CYP79B3, and CYP83B1", BIOTECHNOLOGY AND BIOPROCESS ENGINEERING, KOREAN SOCIETY FOR BIOTECHNOLOGY AND BIOENGINEERING, SEOUL, KR, vol. 14, no. 4, 1 July 2009 (2009-07-01), pages 467 - 473, XP002598327, ISSN: 1226-8372, DOI: 10.1007/S12257-008-0294-Y
- GAUME A ET AL: "Rhizosecretion of recombinant proteins from plant hairy roots", PLANT CELL REPORTS, SPRINGER INTERNATIONAL, DE, vol. 21, no. 12, 1 August 2003 (2003-08-01), pages 1188 - 1193, XP002598291, ISSN: 0721-7714, [retrieved on 20030618], DOI: 10.1007/S00299-003-0660-3

## Description

L'invention concerne un procédé de production de protéines d'intérêt à partir d'une structure végétale.

Les protéines sont des biopolymères d'acides aminés synthétisés par tous les organismes vivants. Elles sont impliquées dans pratiquement tous les aspects de la vie cellulaire. Les enzymes actionnent et régulent le métabolisme, les protéines structurales façonnent la cellule, les protéines de signalisation et les protéines récepteurs permettent d'intégrer les changements environnementaux de la cellule. Actuellement, les protéines sont largement utilisées non seulement à des fins industrielles (enzymes dans la lessive, additifs alimentaires, agents de blanchiment pour le papier...) mais également à des fins médicales (vaccins et allergènes, hormones, anticorps...). Avant le développement de la biologie moléculaire et des outils de la technologies de l'ADN recombinant, la seule source de protéines d'intérêt était l'organisme les produisant lui-même. Par exemple, l'insuline était auparavant purifiée à partir de porcs tandis que l'hormone de croissance humaine était extraite à partir de tissus de cadavres humains. Les principaux inconvénients de ces approches ont été la disponibilité limitée de la matière de départ et la faible teneur en protéine d'intérêt. En outre, le risque de contamination virale des protéines utilisées pour des applications médicales est resté élevé, surtout quand elles ont été extraites de tissus humains. Dans les années 80, la technologie de l'ADN recombinant a fourni des alternatives à ces problèmes en permettant la surproduction de protéines étrangères (protéines recombinantes) dans un organisme hôte donné. L'insuline animale a ainsi été la première protéine recombinante avec une application médicale à être produite dans la bactérie *Escherichia coli.* A l'heure actuelle, les cultures de cellules animales et d'*E*. *coli* sont les deux références pour la bioproduction de protéines recombinantes.

Cependant, les bactéries sont incapables de produire des protéines glycosylées complexes et la culture de cellules animales est un processus assez coûteux qui ne peut exclure le risque de contamination par un virus de l'animal. Des systèmes de bioproduction alternatifs ont ainsi vu le jour au cours des deux dernières décennies, incluant les plantes qui sont considérées comme sûres (pas de risque viral), capables de produire des protéines complexes et bon marché en quantité. Le confinement des systèmes de bioproduction d'origine végétale (dans les serres pour la plante entière ou dans des bioréacteurs pour des cellules végétales) est préférable à la culture de plantes en plein champ. Les racines chevelues sont un exemple d'un tel système de bioproduction confiné car elles peuvent facilement être cultivées dans des bioréacteurs et des clones transgéniques peuvent être obtenus pour n'importe quel gène d'intérêt. Ce système racinaire particulier est généré suite à l'infection de la cellule végétale par *Rhizobium rhizogenes* qui transfère naturellement plusieurs gènes bactériens dans le génome de plante.

*R. rhizogenes* est une bactérie tellurique pathogène des végétaux responsables d'une maladie appelée « hairy root disease ». Cette maladie se caractérise par l'apparition d'un chevelu racinaire au point d'infection par la bactérie.

La capacité à induire la maladie est liée à la présence dans la bactérie d'un plasmide de haut poids moléculaire (ca. 300 Kb) appelé plasmide Ri qui rend les bactéries virulentes.

Le chevelu racinaire résulte du transfert et de l'expression de l'information génétique portée par un fragment du plasmide pathogène, appelé ADN-T (ADN de transfert) et délimité par deux zones appelée bordure droite (RB) et bordure gauche (LB), de la bactérie vers le génome nucléaire de la cellule végétale.

L'ADN-T de *R. rhizogenes* comporte en outre des gènes *aux* responsables de la synthèse de l'auxine, ainsi que des gènes dits *rol.* Il ne comporte aucun gène impliqué dans la synthèse de cytokinine. Après le transfert de cet ADN-T dans une cellule végétale, ces gènes vont détourner le métabolisme cellulaire pour forcer la cellule de la plante infectée (de tige, feuille ...) à suivre un nouveau programme de développement et ainsi à produire massivement de l'auxine. Dès lors la balance hormonale auxine/cytokinine penche en faveur de l'auxine, induisant ainsi une rhizogénèse importante. Cette induction de la formation de racine mène à l'apparition d'un nouveau système racinaire sur le site de l'infection, les racines dites « chevelues », sorte d'amas de racines présentant un surdéveloppement de poils racinaires.

Les souches de *R. rhizogenes* peuvent être génétiquement modifiées afin d'effectuer le transfert d'un gène d'intérêt dans la cellule végétale (codant par exemple une protéine d'intérêt pharmaceutique) et ainsi produire la protéine d'intérêt par l'ensemble des racines chevelues transgéniques générées à partir de la cellule génétiquement modifiée.

Il est à noter que *Agrobacterium rhizogenes* a été renommé *Rhizobium rhizogenes* suite à des remaniements taxonomiques du genre *Agrobacterium* et de la famille des *Rhizobiaceae. Rhizobium rhizogenes* peut ainsi être identifié sous le nom d'Agrobacterium *rhizogenes.*

De nombreuses protéines hétérologues ont été produites dans des tissus de plantes cultivées *in vitro.*

Une protéine hétérologue, également appelée protéine recombinante ou protéine d'intérêt, est une protéine qui n'est pas naturellement synthétisée par l'organisme qui la produit. Le matériel génétique des cellules de cet organisme (bactéries, cellules végétales en culture, cellules animales en culture etc.) a été modifié par recombinaison génétique afin d'y introduire le gène codant la protéine hétérologue pour pouvoir faire exprimer cette protéine dans ledit organisme.

Kittipongpatana et al. (1998) (Production of solasodine by hairy root, callus, and cell suspension cultures of Solanum aviculare Forst, Pant Cell, Tissue and Organ culture 52 :133-143, 1998) ont montré que des cultures de racines chevelues de *Solanum aviculare,* induites par *Rhizobium rhizogenes,* produisaient une quantité plus importante de solasodine, une protéine endogène de cette espèce, par rapport à des cals indifférenciés ou des cultures de cellules en suspension.

Hellwig et al. (2004) (Plant cell cultures for the production of recombinant proteins. Nature Biotechnology, 22:1415-1422) abordent l'utilisation de racines chevelues pour la production de protéines recombinantes et l'augmentation du rendement de cette production par une optimisation du milieu et des conditions de culture, et une optimisation de la purification des protéines recombinantes.

Dans la demande WO 2011/138233, déposée le 28.04.2011 par l'université de Picardie Jules Verne, les auteurs ont mis au point une méthode de production de protéines recombinantes à partir de racines chevelues transgéniques, obtenues par transformation de plants de la famille des *Brassicaceae* avec *Rhizobium rhizogenes* et/ou *A. tumefaciens.*

Xu J. et al. (2012) (Green factory: Plants as bioproduction platforms for recombinant proteins. Biotechnology Advances 30 :1171-1184), font état des différentes classes de protéines recombinantes pouvant être produites par les plantes, des différentes parties de plantes pouvant être utilisées pour la production de protéines recombinantes et de l'expression stable ou transitoires des protéines recombinantes.

Zhang et al. (2014) (Induction and characterization of callus from Psammosilene tunicoides hairy roots. Journal of Chemical and Pharmaceutical Research, 6:1394-1399) ont étudié la production d'une famille de protéines endogènes de *Psammosilene tunicoides,* les saponines, et ont montré que des cals, obtenus par la dédifférenciation de racines chevelues cultivées dans un milieu MS supplémenté d'auxine 2,4-D, étaient capables de produire autant de saponines que des racines chevelues non dédifférenciées en cals, et une production quatre fois plus importante que des cals obtenus à partir d'autres organes végétaux (feuilles, fleurs...).

La présente invention a pour objet un procédé de production de protéine d'intérêt à partir d'émergences de racines latérales apparaissant sur des racines chevelues d'une plante appartenant à la famille des *Brassicaceae,* en milieu liquide contenant au moins une auxine, comprenant les étapes de :
a) transformation d'une plante appartenant à la famille des *Brassicaceae* avec une souche de *Rhizobium* comprenant les gènes *rol,* afin d'obtenir les racines chevelues, et
b) transformation de ladite plante avec un vecteur contenant une cassette d'expression comprenant un gène codant ladite protéine d'intérêt,
les susdites étapes ayant lieu dans un premier milieu de culture, et
c) induction d'émergences de racines latérales sur les racines chevelues en présence d'au moins une auxine en milieu liquide constituant un deuxième milieu de culture liquide, et
d) culture des racines chevelues présentant des émergences de racines latérales qui ne s'allongent pas, et
e) la sécrétion spontanée dans le milieu de la protéine d'intérêt au cours de la culture, et
f) la récupération de la susdite protéine d'intérêt directement dans le deuxième milieu de culture liquide et
g) optionnellement, la récupération de la susdite protéine d'intérêt accumulée dans les tissus par broyage des racines chevelues.

Les inventeurs ont découvert de manière surprenante que la culture de racines chevelues de plantes de la famille des *Brassicaceae,* obtenues suite à l'infection par *R. rhizogenes,* dans un milieu liquide contenant au moins une auxine induisait la disparition des poils racinaires et le développement de structures particulières de forme conique qui sont désignés ci-après par l'expression « émergences de racines latérales » (ERL). Ces structures coniques se développent le long des racines chevelues et présentent des caractères morphologiques qui les rendent notamment solidaires des racines sur lesquelles ils se développent, ce qui les différencient clairement des cals friables qui sont eux, sphériques, homogènes, sans cellules différenciées.

De manière encore plus surprenante, ils ont découvert que lesdites émergences de racines latérales étaient capables de produire des protéines d'intérêt en quantité plus importante que les racines chevelues conventionnelles, c'est-à-dire obtenues habituellement lors d'une infection par *R. rhizogenes.* En effet, ils ont constaté qu'une fluorescence importante, liée à la production de la protéine d'intérêt, était présente dans le milieu gélosé, ce qui suggérait que la protéine diffusait facilement à partir des racines ainsi modifiées.

Les caractéristiques morphologiques des émergences de racines latérales et leur utilisation pour la production d'une protéine d'intérêt constitue l'originalité de cette invention.

**La transformation** par *Rhizobium rhizogenes* est une technique connue dans l'état de la technique. L'homme du métier est familier des différentes techniques couramment utilisées pour la réalisation de ladite étape de transformation. Selon les espèces à transformer, les différentes parties de la plante peuvent être utilisés pour l'infection (hypocotyles, feuilles, etc.).

Généralement, l'infection par *R. rhizogenes* est réalisée en appliquant un inoculum de *R. rhizogenes* sur les tissus végétaux qui ont été précédemment blessés.

Par ***Rhizobium rhizogenes*** on entend également *Agrobacterium rhizogenes,* ancienne appellation de cette espèce bactérienne avant remaniements taxonomiques du genre *Agrobacterium* et de la famille des *Rhizobiaceae.*

Telle qu'utilisée ici, l'expression **"plante appartenant à la famille des *Brassicaceae"*** a le sens général utilisé dans l'état de la technique. Il englobe toute plante de la famille des *Brassicaceae* anciennement connu comme nommée crucifères.

Elle contient plus de 330 genres et environ 3700 espèces, selon les jardins botaniques royaux de Kew. Les plantes appartenant à la famille des *Brassicaceae* sont par exemple le chou, le navet, le colza, la moutarde, le raifort, le cresson, le radis, la roquette, le rutabaga.

Les plus grands genres sont Draba (365 espèces), Cardamine (200 espèces, mais sa définition est controversée), Erysimum (225 espèces), Lepidium (230 espèces) et Alyssum (195 espèces). Les genres bien connus sont par exemple Arabidopsis, Armoracia (genre du raifort), Barbarea (genre du cresson de terre), Brassica (choux, moutarde, navet, chou-rave, colza, rutabaga), Crambe (crambe maritime ou chou marin), Eruca (roquette), Erysinum (giroflée), Raphanus (radis), Nasturtium, Wasabia (wasabi).

Les espèces bien connues sont par exemple *Brassica oleracea* (chou, chou-fleur, etc.), *Brassica rapa* (navet, chou chinois, etc.), *Brassica napus* (colza, etc.), *Raphanus sativus* (radis commun), *Armoracia rusticana* (raifort), *Matthiola logipetala* (giroflée odorante), *Arabidopsis thaliana* (organisme modèle en génétique).

Telle qu'utilisée ici l'expression « **protéine d'intérêt** » correspond à toute protéine pouvant être produite par le procédé selon l'invention. La protéine d'intérêt peut être aussi bien une protéine endogène à la plante, qu'une protéine hétérologue.

Dans le cas où la protéine d'intérêt est une protéine endogène à la plante, c'est-à-dire naturellement produite par la plante, la plante de la famille des *Brassicaceae* modifiée par le procédé selon l'invention, surproduira et sécrétera ladite protéine endogène par rapport à une plante non modifiée.

Dans le cas, où la protéine d'intérêt est une protéine hétérologue, également dite protéine recombinante, la plante modifiée par le procédé selon l'invention produira une protéine habituellement non présente dans la plante appartenant à la famille des *Brassicaceae.*

Cette protéine d'intérêt peut être toute protéine d'origine végétale ou animale, dont les protéines complexes en terme de structure tridimensionnelle, dont les protéines présentant des ponts disulfures ou des associations de chaines ou des sites de glycosylation, en particulier des anticorps monoclonaux ou des enzymes ayant une fonction de type hydrolase, oxydoréductase, transférase, estérase ou des fonctions de transport comme par exemple des sulfatases impliquées dans des maladies rares ou des enzymes digestives.

Le terme « **auxine** » utilisé ici désigne une hormone végétale, ou phytohormone, qui joue un rôle majeur dans le contrôle de la croissance et du développement des plantes. Cette phytohormone intervient dès les premiers stades de l'embryogenèse puis contrôle aussi bien l'organisation du méristème apical (phyllotaxie) et la ramification des parties aériennes de la plante (dominance apicale), que la formation de la racine principale, l'initiation des racines latérales et des racines adventives (rhizogénèse). L'auxine intervient également dans les tropismes en réponse à la gravité (gravitropisme) ou à la lumière (phototropisme). Ces multiples effets à l'échelle de la plante résultent du contrôle qu'elle exerce sur la division cellulaire, l'élongation cellulaire et certaines étapes de différenciation. Il existe différents types d'auxine.

L'expression « **gènes *rol »*** employée ici a le sens général connu de l'art antérieur. Elle fait référence au groupe de gènes bactériens qui sont capables d'induire la formation de racines chevelues (Schmülling et ah, 1988; Boulgakov et ah, 2008). Typiquement, les gènes *rol* sont portés par un plasmide tel qu'un plasmide pRi endogène aux souches de *R. rhizogenes.*

Tel qu'il est utilisé ici, le terme « **cassette d'expression** » a le sens général utilisé dans l'état de la technique. Elle se réfère à une construction d'acide nucléique qui, lorsqu'il est présent dans une cellule donnée, dans des conditions appropriées, permet l'expression d'un gène d'intérêt. Selon la présente invention, ledit gène d'intérêt est le gène codant une protéine d'intérêt que l'on souhaite produire et collecter.

La cassette d'expression comprend un promoteur, éventuellement une séquence codant un peptide signal, un polylinker, et un signal de polyadénylation, le tout dans un plasmide vecteur.

L'expression « **sécrétion spontanée** » dans le milieu de la protéine d'intérêt signifie que ladite protéine d'intérêt produite par les cellules végétales est sécrétée naturellement par l'organisme végétal dans son milieu de culture par son système de sécrétion par défaut, c'est-à-dire par la voie du réticulum endoplasmique et de l'appareil de Golgi.

Les protéines d'intérêt produites dans les racines peuvent être récupérées par broyage des tissus suivi d'une centrifugation pour éliminer la fraction insoluble. La protéine peut ensuite être purifiée de la fraction soluble par des techniques de chromatographie.

Dans un mode de réalisation, le milieu de culture contenant ladite protéine d'intérêt est récupéré et est utilisé directement pour des applications futures.

Dans une variante de réalisation, la protéine d'intérêt est obtenue après une ou plusieurs étapes de purification. Typiquement, le milieu de culture peut d'abord être clarifié par filtration standard ou centrifugation à basse vitesse pour éliminer les débris cellulaires. La protéine d'intérêt est ensuite soit précipitée en utilisant une haute concentration saline puis dialysée, soit directement fixée sur une colonne de chromatographie d'affinité. La protéine d'intérêt concentrée peut alors être lyophilisé ou stockée dans un tampon de stockage approprié à basse température.

Les protocoles adaptés à chaque protéine d'intérêt pouvant être obtenue selon l'invention, et pour chaque type d'application envisagée, sont des techniques standard de l'état de la technique, et l'homme du métier sélectionnera facilement la ou les étape(s) de purification appropriée (s), pour l'application souhaitée.

Un avantage de la présente invention est de simplifier la récupération et le traitement en aval de la protéine d'intérêt. Un autre avantage est que la biomasse de racines n'est pas détruite par la récupération des protéines et une culture donnée peut être utilisée pour plusieurs cycles de production de ladite protéine d'intérêt. Contrairement aux plantes cultivées dans des lieux où les facteurs environnementaux (changements de température, sécheresse, attaques de ravageurs, pesticides / utilisation d'herbicides ...) peuvent considérablement influer sur le niveau de production de la protéine d'intérêt, les conditions de culture des racines transgéniques dans des bioréacteurs sont contrôlées et standardisées, permettant ainsi une production homogène entre les différents lots. De plus, les racines ne produisent pas de pollen et ne peuvent pas survivre à l'extérieur du bioréacteur, ce qui élimine le risque de dissémination du transgène dans l'environnement.

Un autre avantage du procédé selon l'invention réside dans le fait que les lignées provenant de racines chevelues présentent une plus grande stabilité génétique que des lignées cellulaires ou des lignées de cals conventionnelles. Le développement d'émergences de racines latérales ne sera induit par l'ajout d'hormones que pour la production de la protéine d'intérêt, contrairement aux lignées cellulaires ou aux lignées de cals qui doivent être maintenues continuellement sur un milieu riche en hormones, ce qui induit une instabilité génétique.

Un autre avantage du procédé selon l'invention est la possibilité de produire des produits biopharmaceutiques pour une délivrance par voie orale en utilisant des espèces de plantes qui fournissent des racines comestibles généralement trouvé dans le régime alimentaire humain / animal. Il n'est dans ce cas pas nécessaire de purifier la protéine d'intérêt.

L'utilisation d'espèces de plantes comestibles depuis plusieurs centaines d'années dans l'alimentation humaine / animale est une bonne indication de leur nature inoffensive contrairement au tabac, qui a été largement utilisée dans le passé pour ce type d'application, et qui appartient à la famille des Solanacées, dont certaines espèces sont bien connues pour leur capacité à produire des composés potentiellement toxiques (alcaloïdes), tels que la nicotine.

L'utilisation d'un système radiculaire de plante comestible pour la production de protéines d'intérêt thérapeutiques réduit ainsi les problèmes de sécurité sanitaire et alimentaire par rapport à la matière première de départ utilisée, avant la purification des protéines (pas de virus animal).

Ainsi, avantageusement, le procédé selon l'invention permet l'obtention de niveaux élevés de protéines d'intérêt, avec une purification réduite et de plus faibles coûts de traitement en aval. Il permet également l'obtention d'une nouvelle formulation de produits biopharmaceutiques à délivrance par voie orale, réduisant les problèmes de sécurité pour la santé humaine et l'environnement.

Selon un mode de réalisation particulier, la présente invention a pour objet un procédé de production de protéine d'intérêt, à partir d'émergences de racines latérales apparaissant sur des racines chevelues d'une plante appartenant à la famille des *Brassicaceae,* en milieu liquide contenant au moins une auxine, comprenant les étapes de :
a) transformation d'une plante appartenant à la famille des *Brassicaceae* avec une souche de *Rhizobium* comprenant les gènes *rol,* afin d'obtenir les racines chevelues, et
b) transformation de ladite plante avec un vecteur contenant une cassette d'expression comprenant un gène codant ladite protéine d'intérêt, et
c) culture en milieu liquide des racines chevelues obtenues selon l'étape a) et b), les susdites étapes ayant lieu dans un premier milieu de culture, et
d) induction d'émergences de racines latérales sur les racines chevelues en présence d'au moins une auxine en milieu liquide, constituant un deuxième milieu de culture liquide et
e) culture dans le deuxième milieu de culture liquide des racines chevelues présentant des émergences de racines latérales qui ne s'allongent pas, et
f) la sécrétion spontanée dans le deuxième milieu de culture liquide de la protéine d'intérêt au cours de la culture, et
g) la récupération de la protéine d'intérêt directement dans le deuxième milieu de culture liquide, et
h) optionnellement, la récupération de la susdite protéine d'intérêt accumulée dans les tissus par broyage des racines chevelues.

Selon un mode de réalisation particulier, la présente invention a pour objet un procédé de production de protéine d'intérêt tel que décrit ci-dessus dans lequel la souche de Rhizobium est une souche de l'espèce *Rhizobium rhizogenes.*

Selon un mode de réalisation particulier, la présente invention a pour objet un procédé de production de protéine d'intérêt tel que précédemment ci-dessus dans lequel la souche de *Rhizobium rhizogenes* est choisie parmi les souches ATCC 25818, LBA 9402, A4T, A4, LBA1334, ATCC 11325, ATCC 15834 et LMG 155 et préférentiellement choisie parmi la souche ATCC 25818 ou la souche ICPB TR7.

Selon un mode de réalisation particulier, la présente invention a pour objet un procédé de production de protéine d'intérêt tel que décrit précédemment dans lequel dans lequel les étapes a et b sont simultanées.

Selon un mode de réalisation particulier, la présente invention a pour objet un procédé de production de protéine d'intérêt tel que décrit précédemment, dans lequel les étapes a et b sont simultanées, par transformation d'une plante appartenant à la famille des *Brassicaceae* avec une souche de *Rhizobium* comprenant les gènes *rol* et une cassette d'expression comprenant un gène codant ladite protéine d'intérêt, afin d'obtenir les racines chevelues.

Selon un mode de réalisation particulier, la présente invention a pour objet un procédé de production de protéine d'intérêt dans un milieu de culture liquide contenantau moins une auxine, à partir d'émergences de racines latérales qui ne s'allongent pas apparaissant sur des racines chevelues d'une plante appartenant à la famille des *Brassicaceae,* comprenant les étapes de :
a) transformation d'une plante appartenant à la famille des *Brassicaceae* avec une souche de *Rhizobium* comprenant les gènes *rol* et une cassette d'expression comprenant un gène codant ladite protéine d'intérêt afin d'obtenir les racines chevelues, et
b) culture en milieu liquide des racines chevelues obtenues selon l'étape a), les susdites étapes ayant lieu dans un premier milieu de culture, et
c) induction d'émergences de racines latérales sur les racines chevelues en présence d'au moins une auxine en milieu liquide, constituant un deuxième milieu de culture et
d) culture dans le deuxième milieu de culture liquide des émergences de racines latérales qui ne s'allongent pas apparaissant sur les susdites racines chevelues, et
e) la sécrétion spontanée dans le deuxième milieu de culture liquide de la protéine d'intérêt au cours de la culture, et
f) optionnellement, la récupération de la susdite protéine d'intérêt exprimée par extraction des tissus et directement dans le deuxième milieu de culture liquide.

Selon un mode de réalisation particulier, la présente invention a pour objet un procédé de production de protéine d'intérêt tel que décrit ci-dessus, dans lequel l'étape a est effectuée avant l'étape b.

Selon un mode de réalisation particulier, la présente invention a pour objet un procédé de production de protéine d'intérêt tel que décrit précédemment, dans lequel l'étape b est effectuée avant l'étape a.

Selon un mode de réalisation particulier, la présente invention a pour objet un procédé de production de protéine d'intérêt tel que décrit précédemment, dans lequel l'étape d'induction d'émergences de racines latérales sur les racines chevelues en présence d'au moins une auxine en milieu liquide est effectuée par addition d'au moins une auxine dans le premier milieu de culture liquide des racines chevelues, pour donner le deuxième milieu de culture liquide.

Selon un mode de réalisation particulier, la présente invention a pour objet un procédé de production de protéine d'intérêt tel que décrit précédemment, dans lequel l'étape d'induction d'émergences de racines latérales sur les racines chevelues en présence d'au moins une auxine en milieu liquide est effectuée par remplacement d'un premier milieu de culture des racines chevelues par un deuxième milieu de culture liquide contenant au moins une auxine.

Selon un mode de réalisation particulier, la présente invention a pour objet un procédé de production de protéine d'intérêt tel que décrit précédemment, dans lequel l'étape de récupération de la susdite protéine d'intérêt exprimée est effectuée directement dans le deuxième milieu de culture après une sécrétion spontanée au cours de la culture et optionnellement par broyage des tissus cultivés.

Selon un mode de réalisation particulier, la présente invention a pour objet un procédé de production de protéine d'intérêt tel que décrit précédemment, dans lequel l'auxine est choisie parmi : acide 2,4-dichlorophénoxyacétique (2,4-D), acide 3-indoleacétique (IAA), acide indole-3-butyrique (IBA), acide 1-naphtalèneacétique (NAA), acide 2,4,5-trichlorophénoxyacétique (2,4,5-T), acide 2,3,5-triiodoacétique, acide 4-chlorophénoxyacétique, acide 2-naphtoxyacétique, acide 1-naphtylacétique, acide 4-amino-3,5,6-trichloropicolinique, acide 3,6-dichloro-2-méthoxybenzoique (Dicamba).

Selon un mode de réalisation particulier, la présente invention a pour objet un procédé de production de protéine d'intérêt tel que décrit précédemment, dans lequel l'auxine 2,4-D est utilisée à une concentration de 0,01 à 10 mg/l, notamment de 0,2 à 1 mg/l.

Selon un mode de réalisation particulier, la présente invention a pour objet un procédé de production de protéine d'intérêt tel que décrit précédemment, dans lequel l'auxine 2,4-D est utilisée à une concentration de 0,5 mg/l.

Selon un mode de réalisation particulier, la présente invention a pour objet un procédé de production de protéine d'intérêt tel que décrit précédemment, dans lequel l'auxine 2,4-D est utilisée à une concentration de 1 mg/l.

Selon un mode de réalisation particulier, la présente invention a pour objet un procédé de production de protéine d'intérêt tel que décrit précédemment, dans lequel la plante appartenant à la famille des *Brassicaceae* est choisie parmi les genres *Arabidopsis, Armoracia, Barbarea, Brassica, Crambe, Eruca, Raphanus, Wasabia* et *Camelina.*

Selon un mode de réalisation particulier, la présente invention a pour objet un procédé de production de protéine d'intérêt tel que décrit précédemment, dans lequel la plante appartenant à la famille des *Brassicaceae* est *Brassica rapa* ou *Arabidopsis thaliana.*

Selon un mode de réalisation particulier, la présente invention a pour objet un procédé de production de protéine d'intérêt tel que décrit précédemment, dans lequel la cassette d'expression comprend un peptide signal, ledit peptide signal étant préférentiellement placé en amont du gène codant la protéine d'intérêt.

Selon un mode de réalisation particulier, la présente invention a pour objet un procédé de production de protéine d'intérêt tel que décrit précédemment, dans lequel ledit peptide signal est dérivé d'une plante appartenant à la famille des *Brassicaceae* et notamment est un peptide signal provenant de la pectine méthylestérase (PME) codée par le gène Atlg69940 d'*Arabidopsis thaliana* ou un variant de celui-ci.

Selon un mode de réalisation particulier, la présente invention a pour objet un procédé de production de protéine d'intérêt tel que décrit précédemment, dans lequel la cassette d'expression comprend un promoteur, un peptide signal, un gène codant ladite protéine d'intérêt et une séquence de polyadénylation.

Selon un mode de réalisation particulier, la présente invention a pour objet un procédé de production de protéine d'intérêt tel que décrit précédemment, dans lequel ledit promoteur est un promoteur dérivé d'un virus infectant les plantes appartenant à la famille des *Brassicaceae* et notamment le promoteur 35S du virus de la mosaïque du chou-fleur (CaMV).

Selon un mode de réalisation particulier, la présente invention a pour objet un procédé de production de protéine d'intérêt tel que décrit précédemment, dans lequel ledit promoteur peut être remplacé par un autre promoteur inductible par la chaleur ou un élément nutritif.

Selon un mode de réalisation particulier, la présente invention a pour objet un procédé de production de protéine tel que décrit précédemment, dans lequel la protéine produite est une protéine non produite naturellement par le génome de la plante. Il s'agit alors d'une protéine dite d'intérêt.

Selon un mode de réalisation particulier, la présente invention a pour objet un procédé de production de protéine d'intérêt tel que décrit précédemment, dans lequel la protéine d'intérêt est une protéine non produite naturellement par le génome de la plante avant la transformation génétique de ladite plante.

Selon un mode de réalisation particulier, la présente invention a pour objet un procédé de production de protéine tel que décrit précédemment pour une protéine d'intérêt, dans lequel la protéine est une protéine produite naturellement par le génome de la plante avant la transformation génétique de ladite plante mais à un niveau faible.

Selon un mode de réalisation particulier, la présente invention a pour objet un procédé de production de protéine d'intérêt tel que décrit précédemment, dont la modification post traductionnelle de la protéine d'intérêt est une glycosylation ou une phosphorylation.

Selon un mode de réalisation particulier, la présente invention a pour objet un procédé de production de protéine d'intérêt tel que décrit précédemment, dans lequel la séquence de la protéine a été modifiée par au moins une mutation du gène qui la code.

Selon un mode de réalisation particulier, la présente invention a pour objet un procédé de production de protéine d'intérêt tel que décrit précédemment, dans lequel la protéine d'intérêt est une protéine d'origine virale, notamment une protéine du virus de l'hépatite B référencée sous le numéro d'accession SwissProt P03141.3.

Selon un mode de réalisation particulier, la présente invention a pour objet un procédé de production de protéine d'intérêt tel que décrit précédemment, dans lequel la protéine d'intérêt est une protéine d'origine animale, notamment une protéine issue d'un mammifère, ledit mammifère étant choisi parmi les rongeurs, les félins, les canins et les primates.

Selon un mode de réalisation particulier, la présente invention a pour objet un procédé de production de protéine d'intérêt tel que décrit précédemment, dans lequel la protéine d'intérêt est une protéine d'origine humaine.

Selon un mode de réalisation particulier, la présente invention a pour objet un procédé de production de protéine d'intérêt tel que décrit précédemment, dans lequel la protéine d'intérêt est une protéine d'origine végétale, notamment les protéines glycosylées de plantes.

Selon un mode de réalisation particulier, la présente invention a pour objet un procédé de production de protéine d'intérêt tel que décrit précédemment, dans lequel la protéine végétale est la lectine ou la papaïne.

Selon un mode de réalisation particulier, la présente invention a pour objet un procédé de production de protéine d'intérêt tel que décrit précédemment, dans lequel la protéine d'intérêt est choisie parmi les allergènes, les vaccins, les enzymes, les inhibiteurs d'enzyme, les anticorps, les fragments d'anticorps, les antigènes, les toxines, les peptides anti-microbiens, les hormones, les facteurs de croissance, les protéines du sang, les récepteurs, les protéines de signalisation, les composants protéiques d'étalon biomédicaux, les composants protéiques de milieu de culture cellulaire, les protéines de fusion ou marquées, les peptides ou protéines riches en cystéine.

Selon un mode de réalisation particulier, la présente invention a pour objet un procédé de production de protéine d'intérêt tel que décrit précédemment, dans lequel la protéine du sang produite est l'albumine, les facteurs de la coagulation, les immunoglobulines ou la transferrine.

Selon un mode de réalisation particulier, la présente invention a pour objet un procédé de production de protéine d'intérêt tel que décrit précédemment, dans lequel la protéine est une enzyme ayant une fonction de type hydrolase, oxydoréductase, transférase, estérase, ou des enzymes ayant une fonction de transport telle que la sulfatase, ou une enzyme digestive.

Selon un mode de réalisation particulier, la présente invention a pour objet un procédé de production de protéine d'intérêt tel que décrit précédemment, dans lequel la protéine d'intérêt est un anticorps monoclonal.

Selon un mode de réalisation particulier, la présente invention a pour objet un procédé de production de protéine d'intérêt tel que décrit précédemment, dans lequel la protéine d'intérêt présente des sites de glycosylation.

L'expression « site de glycosylation » se rapporte à un acide aminé d'une protéine pouvant présenter une liaison covalente avec un glucide. Les glycosylations les plus communes sont la N-glycosylation et la O-glycosylation. La N-glycosylation correspond à l'addition d'un oligosaccharide ayant à sa base un « N-acétyl-glucosamine » branché sur une asparagine (Asn) contenue dans la séquence Asn-Xaa-Ser/Thr d'une protéine, Xaa étant n'importe quel acide aminé sauf Pro, Ser ou Thr. La O-glycosylation correspond à l'addition de glucides au niveau des résidus -OH de certains acides aminés sérine et thréonine des protéines.

Selon un mode de réalisation particulier, la présente invention a pour objet un procédé de production de protéine d'intérêt tel que décrit précédemment, dans lequel la protéine d'intérêt présente des associations de chaines.

Une association de chaines nécessite soit la synthèse de deux polypeptides dans les racines suivie de leur association, soit la maturation d'une chaine par clivage dans la plante, suivie de l'association des deux chaines produites.

Selon un mode de réalisation particulier, la présente invention a pour objet un procédé de production de protéine d'intérêt tel que décrit précédemment, dans lequel les protéines ou peptides produits sont riches en cystéine et contiennent au moins un pont disulfure.

Selon un mode de réalisation particulier, la présente invention a pour objet un procédé de production de protéine d'intérêt tel que décrit précédemment, dans lequel la protéine d'intérêt est la lipase, la pepsine ou la trypsine.

Selon un mode de réalisation particulier, la présente invention a pour objet un procédé de production de protéine d'intérêt tel que décrit précédemment, dans lequel la protéine d'intérêt est la lipase gastrique humaine.

Selon un mode de réalisation particulier, la présente invention a pour objet un procédé de production de protéine d'intérêt tel que décrit précédemment, dans lequel la protéine d'intérêt est une interleukine.

Selon un mode de réalisation particulier, la présente invention a pour objet un procédé de production de protéine d'intérêt tel que décrit précédemment, dans lequel la protéine d'intérêt est un interféron.

Selon un mode de réalisation particulier, la présente invention a pour objet un procédé de production de protéine d'intérêt tel que décrit précédemment, dans lequel la protéine d'intérêt est la GFP (Green Fluorescent Protein).

Selon un mode de réalisation particulier, la présente invention a également pour objet une protéine d'intérêt telle qu'obtenue par le procédé précédemment décrit, ladite protéine d'intérêt étant choisie parmi les allergènes, les vaccins, les enzymes, les inhibiteurs d'enzyme, les anticorps, les fragments d'anticorps, les antigènes, les toxines, les peptides anti-microbiens, les hormones, les facteurs de croissance, les protéines du sang, les récepteurs, les protéines de signalisation, les composants protéiques d'étalons biomédicaux, les composants protéiques de milieux de culture cellulaire, les protéines de fusion ou marquées, les peptides ou protéines riches en cystéine et particulièrement choisies parmi l'albumine, les facteurs de la coagulation, les immunoglobulines, la transferrine, les sulfatases, les enzymes digestives, les anticorps monoclonaux, les lipases, en particulier la lipase gastrique humaine, la pepsine, la trypsine, les interleukines ou les interférons.

La présente invention a également pour objet un procédé de production d'émergence de racines latérales apparaissant sur des racines chevelues, en milieu liquide contenant au moins une auxine, comprenant les étapes de :
a) transformation d'une plante appartenant à la famille des *Brassicaceae* avec une souche *de Rhizobium* comprenant les gènes *rol,* afin d'obtenir les racines chevelues, et
b) induction d'émergences de racines latérales qui ne s'allongent pas sur les racines chevelues en présence d'au moins une auxine en milieu liquide.

### FIGURES

**Figure 1** : Organisation de l'ADN de transfert (T-DNA) du plasmide pRP49. LB : Bordure Gauche ; RB : Bordure Droite.
**Figure 2** **:** Morphologie de racines portant des émergences de racines latérales, trois semaines après l'induction hormonale par le 2,4-D.
   **A.** Racines en culture. **B.** Racines portant des émergences de racines latérales
**Figure 3** **:** Vues en coupe longitudinale en microscopie optique. Coloration au bleu de toluidine
   **A.** Emergences de racines latérales se développant au niveau du péricycle d'une racine chevelue, deux semaines après induction par le 2,4-D. La flèche indique la zone corticale de la racine en déliquescence.
   **B.** Emergence de racines latérales montrant des files de cellules (flèche) et une couche de cellules épidermiques (ép)..

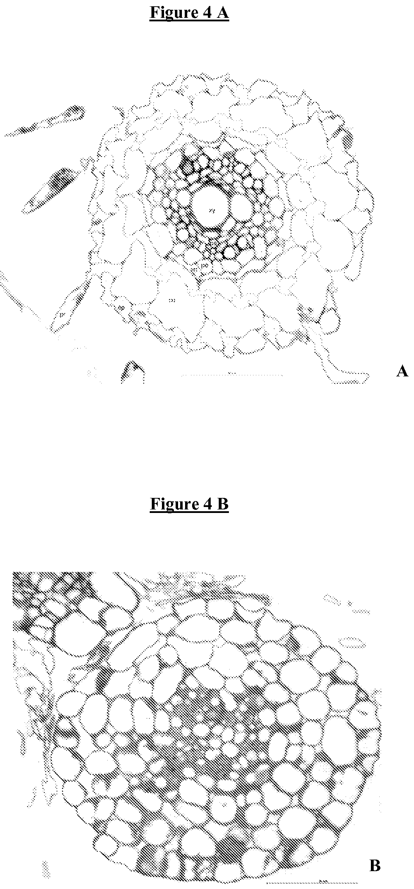

**Figure 4** **:** Vues en coupe transversale en microscopie optique. Coloration au bleu de toluidine
   **A.** Racine chevelue. co, cortex; en, endoderme; ép, épiderme; pa, parenchyme; pé, péricycle; pr, poil racinaire; xy, xylème.
   **B.** Emergence de racines latérales.

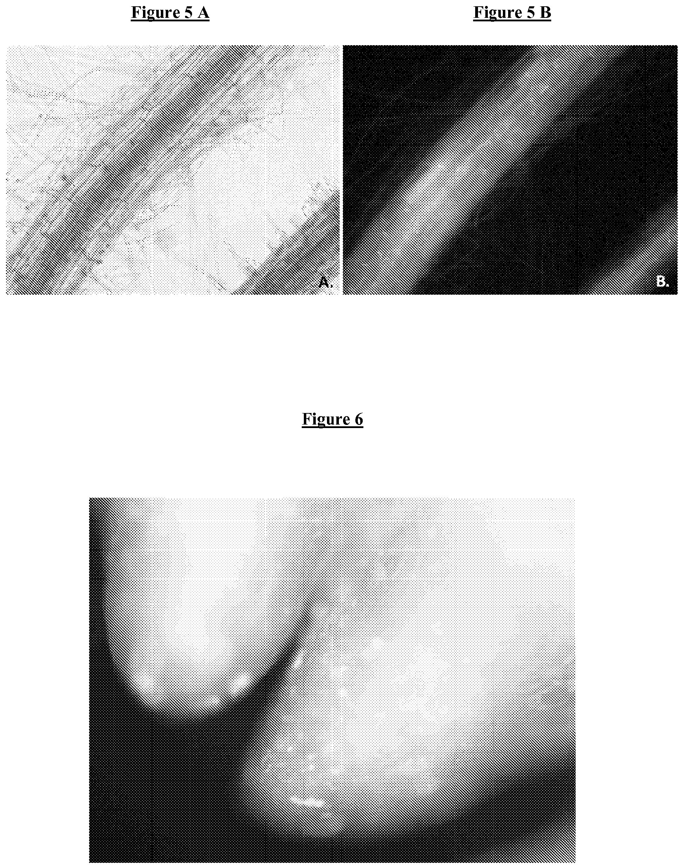

**Figure 5****:** Racines chevelues produisant de la GFP observées au microscope optique.
   **A.** En lumière visible. **B.** En lumière UV
**Figure 6** **:** Emergences de racines latérales contenant la GFP observées en microscopie optique sous lumière UV.

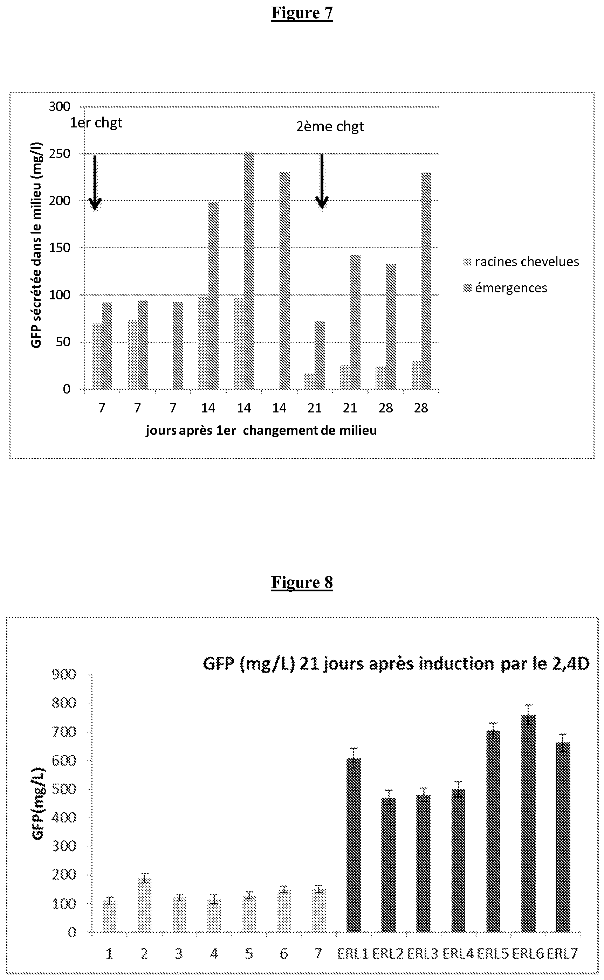

**Figure 7** **:** Concentration en GFP (en mg/l) dans le milieu de culture de racines chevelues (témoins), cultivées dans un milieu sans 2,4-D, et dans le milieu de culture de racines chevelues présentant des émergences de racines latérales, cultivées dans un milieu avec 2,4-D, mesurée à 1 et 2 semaines après un premier renouvellement par un milieu identique neuf, puis à nouveau à 1 et 2 semaines après un second renouvellement par un milieu identique neuf.
**Figure 8** **:** Concentration en GFP (en mg/l) dans le milieu de culture de racines chevelues après 21 jours de culture dans un milieu sans 2,4-D (barres grises 1 à 7) (7 répétitions), et dans un milieu de culture contenant de la 2,4-D à 1 mg/l (barres noires, ERL1 àERL7) (7 répétitions) pour induire le développement d'émergences de racines latérales.

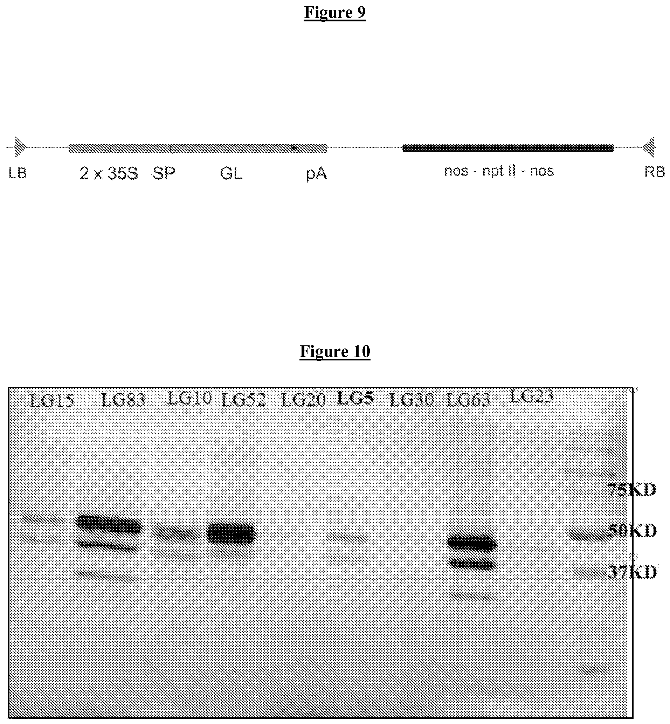

**Figure 9** **:** Organisation de l'ADN de transfert (T-DNA) du plasmide pRP16. LB : Bordure Gauche; RB : Bordure Droite.
**Figure 10** **:** Western blot des clones sécrétant la lipase gastrique humaine

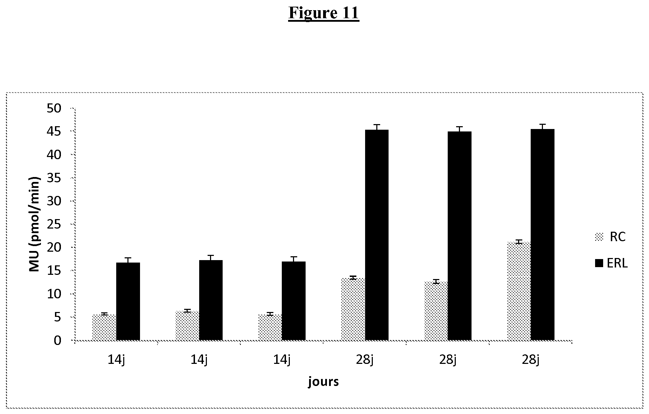

**Figure 11** **:** Activité de la lipase gastrique humaine (pmol/min) après 14 et 28 jours dans le milieu de culture de racines chevelues cultivées dans un milieu sans 2,4-D (barres grises, RC) (3 répétitions), et dans le milieu de culture de racines chevelues cultivées dans un milieu avec 2,4-D (1 mg/l) pour induire le développement d'émergences de racines latérales (barres noires, ERL) (3 répétitions).

### EXEMPLE 1 : Expression du gène gfp

### I. Matériel et Méthodes

### A. Plasmide binaire recombinant pour l'expression de 6XHis-eGFP

La séquence codant la GFP (Green Fluorescent Protein) a été clonée à partir du plasmide pEGFP (Clontech) et insérée dans les sites de restrictions *Nco*I et *EcoRI* de la cassette d'expression pPE45 (Huet et al., 2014), en phase avec la séquence codant le peptide signal du gène At1g69940 d'*Arabidopsis thaliana* et les 6 codons His. Le plasmide résultant, pRP47, a été digéré par *Asp*718 et *Bgl*II et l'ensemble 35S-SP-His-egfp-polyAa été inséré dans les sites *Asp*718*-BamHI* du vecteur binaire pRD400 (Datla et al., 1992), donnant pRP49 (SEQ ID NO : 1). Dans ce plasmide, l'expression du gène *egfp* est réalisée sous le contrôle du promoteur 35S du virus de la mosaïque du chou-fleur (CaMV) dupliqué dans sa séquence activatrice et l'expression du gène *nptII,* conférant la résistance à la kanamycine, est réalisée sous le contrôle du promoteur *nos* (nopaline synthase).

Le plasmide résultant pRP49 ou pRD400-gfp a la séquence suivante prédite SEQ ID NO : 1.

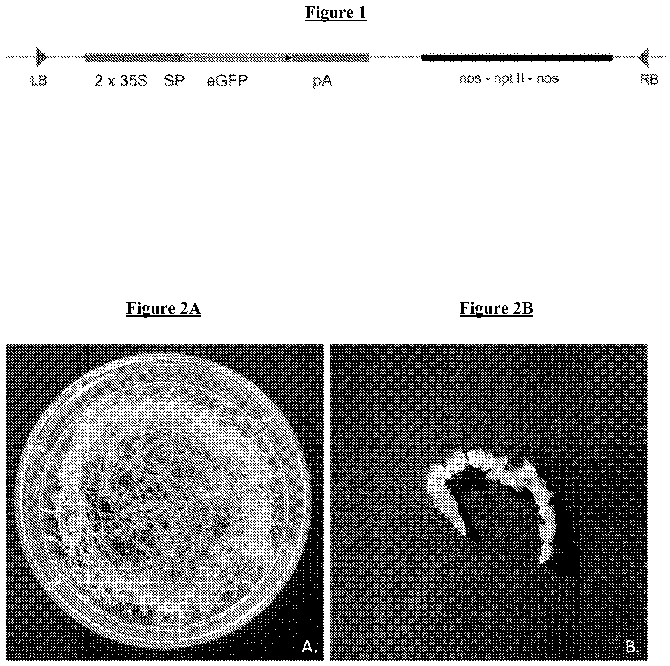

La souche de *Rhizobium rhizogenes* TR7 a été transformée par électroporation avec le plasmide pRP49 (Figure 1).

### B. Production, sélection et mise en culture du clone 2M1

### 1. Espèces de plantes et culture in vitro

Le tableau 1 ci-dessous indique les différentes familles et les espèces de plantes qui ont été testées pour la production de racines chevelues (résultats non montrés).

**Tableau 1 : Familles et espèces de plante testées pour la production de racines chevelues**

| ***5 familles de plantes*** | ***17 espèces de plantes (abréviations)*** |
|---|---|
| *Apiaceae* | *Daucus carota (TOU)* |
| *Asteraceae* | *Lactuca sativa (BRU)* |
| *Brassicaceae* | *Raphanus sativus (CER), Raphanus sativus var. niger (NOI), Brassica Oleracea L. Convar (QUI), Brassica rapa rapa(VER)* |
| *Chenopodiaceae* | *Spinacia oleracea (MAT)* |
| *Solanaceae* | *Nicotiana tabacum (Nt)* |

L'espèce *Brassica rapa rapa* (navet) étant l'espèce pour laquelle les meilleurs résultats de production de racines chevelues ont été obtenus, seule *Brassica rapa rapa* a été utilisée par la suite pour la production de racines chevelues.

Les graines ont été achetées chez Gondian (www.gondian.com).

Leurs surfaces ont été stérilisées avec de l'éthanol à 70% pendant 5 min, puis avec de l'eau de javel à 7% pendant 10 min, et lavées 5 fois avec de l'eau stérile. Les graines ont été placées sur milieu solide Murashige et Skoog dilué au demi (MS/2) à pH 5,8 supplémenté de saccharose à 1%.

La germination et la croissance des semis ont été effectuées à 22°C avec une photopériode de 16h de lumière/8h d'obscurité.

### 2. Infection des plantes par R. rhizogenes

La souche TR7 (ATCC 25818) de *R. rhizogenes* provenant de la collection BCCM^{™}/LMG Laboratorium voor Microbiologie, Université de Gent, a été utilisée.

*R. rhizogenes* a été cultivé dans un milieu liquide MGL (Mannitol, Glutamate, Levure) à pH 7,0 comprenant : 2,5 g/l d'extrait de levure, 5 g/l de tryptone, 5 g/l de mannitol, 5 g/l de NaCl, 1,16 g/l de Na-glutamate, 0,25 g/l de KH₂PO₄, 0,1 g/l MgSO₄, 1,0 mg/l de biotine, 8 g/l Agar, et éventuellement additionné de 50 mg/l de kanamycine pour la sélection du plasmide binaire.

Des inocula ont été préparés à partir de 20 ml de culture bactérienne liquide cultivée sur la nuit à 25°C dans du milieu MGL.

La suspension a été centrifugée pendant 5 min à 15 000 g et les cellules collectées ont été remises en suspension dans du milieu liquide MGL neuf de manière à obtenir une densité optique de 1 ± 0,1 à 600 nm.

L'infection des plants de *Brassica rapa rapa* a été réalisée en piquant des hypocotyles âgés de 3 à 10 jours, obtenus suite à la mise en culture des graines à l'étape B. 1, à l'aide d'une aiguille trempée dans la suspension bactérienne et en essuyant le surplus avec un coton-tige. Le développement de racines chevelues au niveau de la zone blessée a été observé 7 à 10 jours après l'infection.

### 3. Sélection et culture de clones de racines chevelues exprimant la 6XHis-eGFP

Les racines chevelues se développant sur les hypocotyles infectés ont été découpées et placées individuellement sur un milieu de Murashige et Skoog à pH 5,8 contenant 3% de saccharose et 300 mg/L de céfotaxime (MS3cef). Après 7 à 10 jours, 40 pointes de racines chevelues indépendantes (clones) ont été transférées sur un milieu liquide MS3cef où elles ont été cultivées pendant 2 à 4 semaines, à 23°C à l'obscurité

Pour chaque clone, un fragment a été directement contrôlé en utilisant un microscope Nikon Eclipse 90i pour observer l'émission de fluorescence due à la présence de GFP.

Les images ont été prises à l'aide de la caméra Nikon Digital Sight DS-5Mc.

Les 10 clones présentant le plus de fluorescence ont été sélectionnés pour être cultivés en milieu liquide.

Pour initier les cultures en milieu liquide, un fragment d'environ 1 cm de chaque clone de racine chevelue a été transféré dans une boite de Petri contenant 5 ml de milieu liquide Gamborg B5 (Duchefa) pH 5,8 additionné de 3% de saccharose et de 300 mg/l de cefotaxime (B53cef). Les fragments ont ensuite été cultivés pendant 10 jours à 23°C à l'obscurité en agitation à 56 RPM.

Les racines chevelues ont été ensuite successivement cultivées dans une fiole d'Erlenmeyer de 100 ml contenant 20 ml de B53cef pendant 3 semaines, puis dans une fiole d'Erlenmeyer de 250 ml contenant 100 ml de B53cef à nouveau pendant 3 semaines à 26°C sous une faible luminosité en agitation à 110 RPM.

Après ces étapes visant à éliminer les *Rhizobium* par l'utilisation de l'antibiotique cefotaxime, les conditions standards de culture ont été les suivantes : 100 ml de milieu liquide Gamborg B5 additionné de 3% de saccharose, pH 5,8 à 26°C sous une faible luminosité, sur un agitateur Gerhardt RO20 à 110 rpm. Les différents clones de racines ont été repiqués toutes les 3 semaines en utilisant 1 g de biomasse de racines pour 100 ml de milieu de culture liquide Gambord B5 additionnés de 3% de saccharose.

Le clone de *Brassica rapa rapa* présentant les meilleures performances d'émission de fluorescence a été sélectionné et nommé clone 2M1.

### C. Induction d'émergences de racines latérales sur des racines chevelues du clone 2M1

### 1. Induction d'émergences de racines latérales (ERL)

Pour modifier la morphologie des racines chevelues et empêcher le développement des poils racinaires, des racines du clone 2M1 produisant la GFP ont été cultivées pendant deux semaines en milieu liquide B53. Ce milieu de culture a ensuite été éliminé et remplacé par du milieu neuf B53 contenant de l'acide 2,4-dichlorophénoxyacétique (2,4-D), hormone de type auxine, à 0,5 mg/l.

De nombreuses structures coniques, nommées « émergences de racines latérales » (ERL) se sont alors développées sur les racines chevelues (figure 2A) au cours des 7 jours suivants. Les « émergences de racines latérales » se présentent sous la forme de boursouflures sur chaque racine (figure 2B). Ces émergences persistent par la suite sans autres modifications, c'est-à-dire que la présence d'auxine 2,4-D dans le milieu après l'apparition de ces émergences latérales, empêche la différenciation de ces émergences en racines.

### 2. Observation en microscopie optique (comparaison lumière visible et UV)

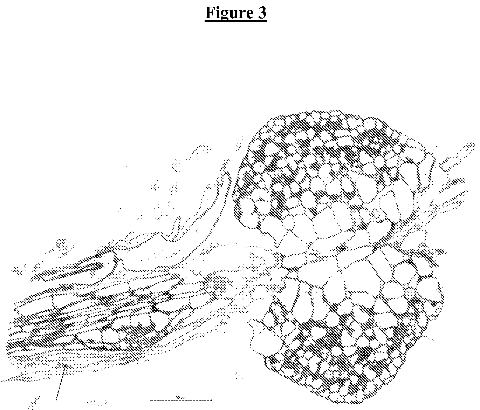

Pour caractériser ces structures coniques ou « émergences de racines latérales » se développant sur les racines après induction hormonale, des coupes longitudinales (figure 3) et transversales (figure 4) ont été réalisées dans des racines chevelues témoins après 2 semaines de culture en milieu B53 et dans des racines chevelues présentant des émergences de racines latérales après 2 semaines de culture en milieu B53 additionné d'auxine 2,4-D.

Les tissus ont été fixés dans de la p-formaldéhyde à 4% pendant une nuit à 4°C, déshydratés dans des bains d'éthanol de concentrations croissantes, puis inclus dans de la résine LR White. Des coupes de 0,5 à 1 µm d'épaisseur ont été réalisées sur un microtome Leica RM2265, colorées au bleu de toluidine et observées sur un microscope Nikon Eclipse 90i. Les images ont été obtenues à l'aide d'un appareil Nikon Digitak Sight DS-5Mc, en lumière visible ou en fluorescence pour localiser la GFP.

### 3. Quantification par fluorométrie de la 6XHis-eGFP libérée dans le milieu de culture par des racines chevelues présentant des émergences de racines latérales

Pour la quantification de 6XHis-EGFP, du milieu de culture a été dilué 20x ou 40x dans un tampon tris à 50 mM et pH 7,5. La fluorescence de la solution a été mesurée dans le fluoromètre BioRad VersaFluor ^{™} (filtre d'excitation 485-495 nm ; filtre d'émission 505-515 nm). La calibration du fluoromètre a été réalisée à l'aide de la 6XHis-EGFP recombinante disponible dans le commerce (BioVision) à une concentration de 10 mg/l.

### II. Résultats

### 1. Observations microscopiques

L'observation en lumière visible des coupes de racines chevelues témoins, c'est-à-dire obtenues dans du milieu de culture sans 2,4-D, fait apparaître les tissus typiques des racines de dicotylédones, à partir de la périphérie: une couche de cellules épidermiques, une zone de cortex, une couche de cellules endodermiques, un péricycle et un cylindre central contenant les faisceaux conducteurs dans du parenchyme (figure 4A).

L'organisation des racines chevelues, obtenues après culture en présence d'auxine 2,4-D, est très différente. La zone corticale a entièrement dégénéré et des boursouflures sont observées au niveau du péricycle. Ces renflements semblent être à l'origine des structures coniques, émergences de racines latérales, se développant sur les racines chevelues.

Les coupes longitudinales (figures 3A et 3B) et transversales (figure 4B) réalisées dans ces structures coniques montrent des tissus hétérogènes dont certains rappellent ceux présents dans les racines: couche de cellules épidermiques, ébauche de cylindre central contenant des cellules plus denses (figure 4B). Le développement de ces structures n'est pas anarchique: des files de cellules sont observées (figure 3A), indication de divisions cellulaires orientées comme c'est le cas dans des pointes racinaires. Il s'agit donc d'émergences de racines latérales à partir de certaines cellules du péricycle (figure 3A).

Ces caractères morphologiques différentient clairement ces structures coniques ou émergences de racines latérales, des cals friables qui sont eux, sphériques, homogènes, sans cellules différenciées (Ikeuchi et al (2013) « Plant Callus: Mechanisms of Induction and Repression » Plant Cell 25 :3159-3173).

L'observation microscopique en lumière visible permet de montrer que la transformation de cellules de plantes par *R. rhizogenes* résulte en la production de racines très ramifiées présentant d'innombrables poils racinaires et ainsi nommées racines chevelues témoins (figure 5A).

Des observations au microscope en épi-fluorescence de racines chevelues témoins exprimant la GFP ont montré que les poils racinaires, bien qu'occupant un grand volume, ne produisaient pas ou peu de protéine (figure 5B). D'autre part, les mêmes observations au microscope ont montré d'une part que les cellules des racines âgées de 4 semaines contenaient peu de GFP et d'autre part, que la GFP se retrouvait essentiellement au niveau du cylindre central (figure 5B). Cette observation laisse supposer qu'il y aurait moins de production et moins de diffusion dans le milieu des protéines d'intérêt lorsqu'elles sont produites par des racines âgées.

A contrario, l'observation des racines chevelues présentant des émergences de racines latérales au microscope à épi-fluorescence a révélé une fluorescence importante (figure 6).

### 2. Quantification de la sécrétion de la GFP

### a. Test 1

Après deux semaines de culture de racines chevelues du clone 2M1 en milieu liquide B53, ce milieu a été remplacé par du milieu B53 neuf contenant de l'auxine 2,4-D à 0,5 mg/l, afin d'induire le développement des émergences de racines latérales. Les racines chevelues du clone 2M1 ont ainsi été cultivées dans des boites de Petri contenant 5 ml de milieu B53 additionné de 2,4-D, à 23°C et sous une agitation de 56 rpm.

La GFP sécrétée dans le milieu de culture par les racines chevelues présentant des émergences de racines latérales, a été quantifiée (10 répétitions) à une et deux semaines après l'ajout d'auxine 2,4-D à 0,5mg/l (tableau 2).

**Tableau 2: Quantification de la GFP en mg/l dans le milieu de culture d'émergences de racines latérales à 1 et 2 semaines après le remplacement du milieu de culture B53 par un milieu de culture B53 additionné d'auxine 2,4-D.**

| Boite de Pétri (5mL B53) | 1 semaine après B53 + 2,4-D | 2 semaines après B53 + 2,4-D |
|---|---|---|
| 1 | 57 | 190 |
| 2 | 58 | 185 |
| 3 | 55 | 167 |
| 4 | 48 | 144 |
| 5 | 49 | 152 |
| 6 | 51 | 158 |
| 7 | 49 | 159 |
| 8 | 59 | 169 |
| 9 | 47 | 153 |
| 10 | 50 | 161 |
| Moyennes clones | 52 | 164 |
| Intervalle de confiance (p=0.95) | 3,2 | 10,3 |

Des racines chevelues du clone 2M1 ont été cultivées (9 répétitions) dans les mêmes conditions avec un changement du milieu B53 par du milieu B53 neuf mais sans ajout d'auxine après les deux premières semaines de culture, afin de constituer des lots de racines chevelues témoins.

La GFP sécrétée dans le milieu de culture par les racines chevelues conventionnelles (témoins) a été quantifiée une et deux semaines après le remplacement par du milieu B53 neuf sans 2,4-D (Tableau 3).

**Tableau 3: Quantifications de la GFP (en mg/l) dans le milieu de culture de racines chevelues témoins à une et deux semaines après le remplacement du milieu de culture B53 par un milieu de culture B53 neuf (9 répétions).**

| | 1 semaine après changement de milieu B53 | 2 semaines après changement de milieu B53 |
|---|---|---|
| 1 | 48 | 123 |
| 2 | 50 | 150 |
| 3 | 58 | 146 |
| 4 | 46 | 159 |
| 5 | 36 | 168 |
| 6 | 40 | 161 |
| 7 | 32 | 153 |
| 8 | 25 | 128 |
| 9 | 22 | 143 |
| Moyennes | 40 | 148 |
| Intervalle de confiance | 9,2 | 11,5 |

La concentration en GFP dans le milieu de culture des racines chevelues du clone 2M1, deux semaines après l'ajout d'auxine 2,4-D était de 164 ± 10,3 mg/l (n=10).

La concentration en GFP dans le milieu de culture des racines chevelues témoins du clone 2M1, deux semaines après le remplacement du milieu B53 par du milieu B53 neuf, était de 148 ± 11,5 mg/l (n=9). Cette production par les racines supérieure à ce qui a été publié (~120 mg/l ; Huet *et al.,* 2014) est due au renouvellement du milieu après deux semaines de culture. La comparaison par un test de Student des résultats obtenus pour les racines chevelues du clone 2M1 cultivées en présence de 2,4-D, et présentant alors des émergences de racines latérales, par rapport à ceux obtenus pour les racines chevelues témoins, font apparaitre une différence significative (p< 0,05) : une quantité plus importante de GFP est retrouvée dans le milieu de culture des racines chevelues portant les émergences de racines latérales que dans le milieu des racines chevelues témoins, c'est-à-dire sans émergences de racines latérales.

### b. Test 2

Le clone 2M1 a été inoculé à t0 dans 5 fioles d'Erlenmeyer à raison 2 g de racines fraiches dans 100 ml de milieu B53. Après une culture de 15 jours à 26°C sur un agitateur à 110 rpm, les racines ont atteint une masse de 24,4 g +/- 3,90.

Un premier changement de milieu a alors été effectué dans chacune des fioles de la manière suivante :
- dans deux fioles, le milieu B53 a été remplacé par du milieu B53 neuf afin de réaliser deux répétitions pour les racines chevelues témoins ;
- dans trois fioles, le milieu B53 a été remplacé par du milieu B53 additionné de 2,4- D à 1 mg/l afin de réaliser trois répétitions pour l'induction du développement d'émergences de racines latérales.

La GFP sécrétée dans le milieu de culture a été quantifiée à 7 jours et à 14 jours après ce premier changement de milieu.

Un second changement de milieu a ensuite été effectué dans ces mêmes fioles de manière identique :
- dans les deux fioles de racines chevelues témoins, le milieu B53 a été remplacé par du milieu B53 neuf,
- dans les trois fioles de racines chevelues induites, le milieu B53 additionné de 2,4-D à 1 mg/l a été remplacé par du milieu B53 additionné de 2,4-D à 1 mg/l neuf.

L'ensemble des résultats est présenté dans la figure 7.

Dans ces conditions, la sécrétion de GFP par les cultures traitées au 2,4-D est doublée après 14 jours de traitement. Cette observation est accentuée après le second renouvellement de milieu de culture, où la sécrétion est augmentée d'un facteur 5 à 9.

### c. Test 3

Des racines du clone 2M1 ont été inoculées dans 14 boites de Petri contenant chacune 5 ml de milieu de culture liquide B53. Elles ont été cultivées pendant 17 jours à 26°C sous une agitation de 110 rpm.

Le milieu a ensuite été changé dans les boites de Petri de la manière suivante :
- dans sept boites de Petri le milieu a été éliminé et remplacé par du milieu de culture B53 neuf additionné de 2,4-D à 1 mg/l (ERL 1 à 7) afin d'induire le développement d'émergences de racines latérales dans ces boites;
- et dans les sept autres boites de Petri le milieu a été éliminé et remplacé par du milieu de culture B53 neuf (Témoins 1 à 7).

Les racines ont été maintenues pendant 21 jours à 26°C sous une agitation de 110 rpm.

La figure 8 présente les concentrations en GFP dans le milieu de culture, après ces 21 jours de culture.

Après 21 jours, la concentration en GFP dans le milieu des racines présentant des émergences de racines latérales induites par la présence de 2,4-D dans le milieu de culture, est 4,3 fois supérieure (597 mg/l +/- 116) à celle dans le milieu de culture de racines chevelues témoins c'est-à-dire sans émergences de racines latérales (138 mg/l +/- 28).

### EXEMPLE 2 : Expression du gène de la lipase gastrique humaine

### I. Matériel et Méthodes

### A. Plasmide binaire recombinant pour l'expression de 6XHis-lipase

La séquence du gène codant la lipase gastrique humaine (GL) a été synthétisée par l'entreprise DNA2.0 sous la forme d'une fusion avec la séquence codant le peptide signal du gène *At1g69940 d'Arabidopsis (SP).* La séquence hybride a ensuite été insérée dans la cassette d'expression pJIT163 (Guerineau *et al.,* 1992), dans les sites de restriction *Nco*I et *EcoR*I, pour donner le plasmide pRP9. L'ensemble 2x35S-SP-GL-polyA contenu dans un fragment du plasmide pRP9 bordé par les sites de restriction *Kpn*I*-Bgl*II a été ensuite inséré aux sites *Kpn*I-*Bam*HI du plasmide binaire pRD400, pour créer le plasmide pRP16 (figure 9).

Le gène synthétisé *GL-SP* a la séquence suivante SEQ ID NO : 2.

La souche TR7 de *Rhizobium rhizogenes* a ensuite été transformée par électroporation (2.5 kV, pendant environ 5 msec) avec le plasmide pRP16.

### B. Production, sélection et mise en culture du clone LG5

### 1. Espèces de plantes et culture in vitro

L'espèce *Brassica rapa rapa* (navet) a été utilisée pour la production de racines chevelues selon le même protocole que précédemment (Exemple 1 partie I.B. 1.).

Les graines ont été achetées chez Gondian (www.gondian.com).

Leurs surfaces ont été stérilisées avec de l'éthanol à 70% pendant 5 min, puis avec de l'eau de javel à 7% pendant 10 min, et lavées 5 fois avec de l'eau stérile. Les graines ont été placées sur milieu solide Murashige et Skoog dilué au demi (MS/2) à pH 5,8 supplémenté de saccharose à 1%.

La germination et la croissance des semis ont été effectuées à 22°C avec une photopériode de 16h de lumière/8 h d'obscurité.

### 2. Infection des plantes par R. rhizogenes

La souche TR7 (ATCC 25818) de *R. rhizogenes* provenant de la collection BCCM^{™}/LMG Laboratorium voor Microbiologie, Université de Gent, a été utilisée.

*R. rhizogenes* a été cultivé dans un milieu MGL liquide à pH 7,0 composé de 2,5 g/l d'extrait de levure, 5 g/l de tryptone, 5 g/l de mannitol, 5 g/l de NaCl, 1,16 g/l de Na-glutamate, 0,25 g/l de KH₂PO₄, 0,1 g/l MgSO₄, 1,0 mg/l de biotine, 8 g/l Agar, et éventuellement additionné de 50 mg/l de kanamycine pour la sélection du plasmide binaire.

Des inocula ont été préparés à partir de 1.5 ml de culture bactérienne liquide cultivée une nuit à 28°C dans du milieu liquide MGL.

La suspension a été centrifugée pendant 1 min à 10 000 g et les cellules collectées ont été remises en suspension dans 100 µl de milieu liquide Gamborg B5 (Duchefa) à pH 5,7 contenant 1 % de saccharose.

L'infection des plants de *Brassica rapa rapa* a été réalisée en piquant des hypocotyles âgés de 10 jours, obtenus suite à la mise en culture des graines à l'étape I. B. 1. (Exemple 2), à l'aide d'une aiguille trempée dans la suspension bactérienne et en essuyant le surplus avec un coton-tige.

L'émergence de racines chevelues au niveau de la zone blessée a été observée 7 à 15 jours après l'infection.

### 3. Sélection et culture de clones de racines chevelues exprimant la 6XHis-lipase

Les racines chevelues se développant sur les hypocotyles infectés ont été découpées et placées sur un milieu solide Gamborg B5 contenant 3% de saccharose et 300 mg/l de céfotaxime. Après 7 à 10 jours de culture, 15 à 40 pointes de racines chevelues indépendantes (clones) ont été transférées sur un milieu liquide Gamborg B5 contenant 3% de saccharose et 300 mg/l de céfotaxime où elles ont été cultivées pendant 2 à 4 semaines.

Elles ont été ensuite cultivées et repiquées régulièrement toutes les 3 semaines dans le même milieu neuf pendant 3 mois jusqu'à la suppression du céfotaxime.

Le clone de chevelu racinaire est alors considéré comme stabilisé et est entretenu par repiquage successif toutes les 3 semaines dans un milieu liquide Gamborg B5 contenant 3% de saccharose.

La présence de lipase gastrique humaine, dans le milieu de culture liquide des racines utilisées pour cette étude, été confirmée par western blot en utilisant un anticorps monoclonal anti-lipase gastrique humaine (Figure 10).

Les milieux de culture des clones sont récupérés puis concentrés 10 fois avec des filtres Amicon (10K). Une migration sur gel de 10 µl de milieu concentré a été réalisée par électrophorèse en condition dénaturante. Après la migration, une empreinte de ce gel a été effectuée par transfert sur une membrane de nitrocellulose. La lipase gastrique humaine a été identifiée sur la membrane de nitrocellulose par méthode de Western Blotting en utilisant successivement un premier anticorps monoclonal anti-lipase gastrique humaine (wh0008513M1, Sigma), puis un deuxième anticorps couplé à la peroxydase (A9044, Sigma) dirigé contre le premier anticorps. La lipase gastrique humaine est alors révélée grâce à l'apparition d'un produit coloré issu de la réaction entre la peroxydase avec les substrats Diaminobenzidine et urée/H₂O₂.

Le clone LG5 a été sélectionné pour son taux de sécrétion le plus élevé.

### C. Induction d'émergences de racines latérales sur les racines chevelues de LG5 et leur caractérisation

### 1. Induction d'émergences de racines latérales sur des racines chevelues

Pour cette étude, on réalise un repiquage dans six fioles d'Erlenmeyer vides dans lesquelles on ajoute 1 g de biomasse d'un même amas de racines du clone LG5 et 100 ml de milieu liquide Gamborg B53. Les racines sont cultivées pendant deux semaines à 26°C et sous agitation à 110 rpm.

Afin d'induire le développement d'émergences de racines latérales (ERL), dans trois fioles d'Erlenmeyer, ce milieu liquide est remplacé, par du milieu de culture liquide neuf B53 contenant de l'acide 2,4-dichlorophénoxyacétique (2,4-D) à 1 mg/l.

Dans les trois autres fioles d'Erlenmeyer présentant des racines chevelues conventionnelles (RC), il est remplacé par du milieu de culture liquide neuf B53, sans 2,4-D.

Après deux semaines de culture à 26°C avec une agitation à 110 RPM, un second changement de milieu a été effectué dans les fioles de manière identique afin de remplacer le milieu de culture par un milieu de culture neuf avec + / - 2,4-D à 1 mg/l.

### 2. Quantification de l'activité de la lipase gastrique

L'activité de la lipase gastrique humaine est mesurée directement à partir des milieux de culture liquides des 6 fioles (14 jours et 28 jours après induction). L'activité enzymatique est mesurée à 37°C en utilisant comme substrat le 4-methylumbelliféryl oléate (0.1 mM). La fluorescence du 4-méthylumbelliférone (MU), produit de la réaction enzymatique par la lipase gastrique, est mesurée avec le fluorimètre (Excitation : 330 nm ; Emission : 450 nm). Cette fluorescence est corrélée à l'activité de la lipase gastrique humaine (figure 11).

### II. Résultats

L'activité de la lipase gastrique humaine a été mesurée dans les milieux de culture liquides des racines chevelues présentant des émergences des racines latérales (ERL1, ERL2 et ERL3) et des racines chevelues conventionnelles (RC1, RC2 et RC3). Cette activité est mesurée à 14 et 28 jours après induction par ajout de 2,4-D (figure 11).

A 14 jours, dans les Erlenmeyers dont le milieu n'a pas été supplémenté en 2,4-D, et contenant des racines chevelues conventionnelles (RC) (témoins), l'activité de la lipase gastrique humaine est de 6 ± 0.3 pmol/min (MU).

En revanche, dans les Erlenmeyers dont le milieu a été supplémenté de 2,4-D, et contenant des émergences de racines latérales (ERL), l'activité de la lipase gastrique humaine est de 16 ± 0.6 pmol/min.

A 28 jours, dans les Erlenmeyers contenant des racines conventionnelles (témoins), l'activité de la lipase gastrique humaine est de 17 ± 0.3 pmol/min, tandis que dans les Erlenmeyers contenant des émergences de racines latérales, l'activité de la lipase gastrique humaine est de 45 ± 0.3 pmol/min.

Cette étude montre que l'activité de la lipase gastrique humaine mesurée dans le milieu liquide des émergences de racines latérales (ERL) est environ 3 fois supérieure à celle mesurée avec des racines chevelues conventionnelles (RC).

## Revendications

1. Procédé de production de protéine d'intérêt à partir d'émergences de racines latérales apparaissant sur des racines chevelues d'une plante appartenant à la famille des *Brassicaceae,* en milieu liquide contenant au moins une auxine, comprenant les étapes de :
a) transformation d'une plante appartenant à la famille des *Brassicaceae* avec une souche de *Rhizobium* comprenant les gènes *rol,* afin d'obtenir les racines chevelues, et
b) transformation de ladite plante avec un vecteur contenant une cassette d'expression comprenant un gène codant ladite protéine d'intérêt,
les susdites étapes ayant lieu dans un premier milieu de culture, et
c) induction d'émergences de racines latérales sur les racines chevelues en présence d'au moins une auxine en milieu liquide constituant un deuxième milieu de culture liquide, et
d) culture des racines chevelues présentant des émergences de racines latérales qui ne s'allongent pas, et
e) la sécrétion spontanée dans le milieu de la protéine d'intérêt au cours de *la* culture, et
f) la récupération de la susdite protéine d'intérêt directement dans le deuxième milieu de culture liquide et
g) optionnellement, la récupération de la susdite protéine d'intérêt accumulée dans les tissus par broyage des racines chevelues.

2. Procédé de production de protéine d'intérêt, à partir d'émergences de racines latérales apparaissant sur des racines chevelues d'une plante appartenant à la famille des *Brassicaceae,* en milieu liquide contenant au moins une auxine, selon la revendication 1, comprenant les étapes de :
a) transformation d'une plante appartenant à la famille des *Brassicaceae* avec une souche de *Rhizobium* comprenant les gènes *rol,* afin d'obtenir les racines chevelues, et
b) transformation de ladite plante avec un vecteur contenant une cassette d'expression comprenant un gène codant ladite protéine d'intérêt, et
c) culture en milieu liquide des racines chevelues obtenues selon l'étape a) et b),
les susdites étapes ayant lieu dans un premier milieu de culture, et
d) induction d'émergences de racines latérales sur les racines chevelues en présence d'au moins une auxine en milieu liquide, constituant un deuxième milieu de culture liquide et
e) culture dans le deuxième milieu de culture liquide des racines chevelues présentant des émergences de racines latérales qui ne s'allongent pas, et
f) la sécrétion spontanée dans le deuxième milieu de culture liquide de la protéine d'intérêt au cours de la culture, et
g) la récupération de la protéine d'intérêt directement dans le deuxième milieu de culture liquide, et
h) optionnellement, la récupération de la susdite protéine d'intérêt accumulée dans les tissus par broyage des racines chevelues.

3. Procédé de production de protéine d'intérêt selon les revendications 1 et 2, dans lequel la souche de Rhizobium est une souche de l'espèce *Rhizobium rhizogenes,* notamment dans lequel ladite souche de *R. rhizogenes* est choisie parmi les souches ATCC 25818, ICPB TR7, LBA 9402, A4T, A4, LBA1334, ATCC 11325, ATCC 15834 et LMG 155 et est préférentiellement choisi parmi la souche ATCC 25818 ou la souche ICPB TR7.

4. Procédé de production de protéine d'intérêt selon les revendications 1 à 3, dans un milieu de culture liquide contenant au moins une auxine, à partir d'émergences de racines latérales qui ne s'allongent pas apparaissant sur des racines chevelues d'une plante appartenant à la famille des *Brassicaceae,* comprenant les étapes de :
a) transformation d'une plante appartenant à la famille des *Brassicaceae* avec une souche de *Rhizobium* comprenant les gènes *rol* et une cassette d'expression comprenant un gène codant ladite protéine d'intérêt afin d'obtenir les racines chevelues, et
b) culture en milieu liquide des racines chevelues obtenues selon l'étape a),
les susdites étapes ayant lieu dans un premier milieu de culture, et
c) induction d'émergences de racines latérales sur les racines chevelues en présence d'au moins une auxine en milieu liquide, constituant un deuxième milieu de culture et
d) culture dans le deuxième milieu de culture liquide des émergences de racines latérales qui ne s'allongent pas apparaissant sur les susdites racines chevelues, et
e) la sécrétion spontanée dans le deuxième milieu de culture liquide de la protéine d'intérêt au cours de la culture, et
f) optionnellement, la récupération de la susdite protéine d'intérêt exprimée par extraction des tissus et directement dans le deuxième milieu de culture liquide.

5. Procédé de production de protéine d'intérêt selon les revendications 1 à 4, dans lequel l'étape d'induction d'émergences de racines latérales sur les racines chevelues en présence d'au moins une auxine en milieu liquide est effectuée par addition d'au moins une auxine dans le premier milieu de culture liquide des racines chevelues, pour donner le deuxième milieu de culture liquide.

6. Procédé de production de protéine d'intérêt selon les revendications 1 à 5, dans lequel l'étape d'induction d'émergences de racines latérales sur les racines chevelues en présence d'au moins une auxine en milieu liquide est effectuée par remplacement d'un premier milieu de culture des racines chevelues par un deuxième milieu de culture liquide contenant au moins une auxine.

7. Procédé de production de protéine d'intérêt selon les revendications 1 à 6, dans lequel l'étape de récupération de la susdite protéine d'intérêt exprimée est effectuée directement dans le deuxième milieu de culture après une sécrétion spontanée au cours de la culture et optionnellement par broyage des tissus cultivés.

8. Procédé de production de protéine d'intérêt selon les revendications 1 à 7, dans lequel l'auxine est choisie parmi :acide 2,4-dichlorophénoxyacétique (2,4-D), acide 3-indoleacétique (IAA), acide indole-3-butyrique (IBA), acide 1-naphtalèneacétique (NAA), acide 2,4,5-trichlorophénoxyacétique (2,4,5-T), acide 2,3,5-triiodoacétique, acide 4-chlorophénoxyacétique, acide 2-naphtoxyacétique, acide 1-naphtylacétique, acide 4-amino-3,5,6-trichloropicolinique, acides 3,6-dichloro-2-méthoxybenzoique (Dicamba).

9. Procédé de production de protéine d'intérêt selon les revendications 1 à 8, dans lequel l'auxine 2,4-D est utilisée à une concentration de 0,01 à 10 mg/l, notamment de 0,2 à 1 mg/l, notamment 0,5 mg/l, notamment 1 mg/l.

10. Procédé de production de protéine d'intérêt selon les revendications 1 à 9, dans lequel la plante appartenant à la famille des *Brassicaceae* est choisie parmi les genres *Arabidopsis, Armoracia, Barbarea, Brassica, Crambe, Eruca, Raphanus, Wasabia* et *Camelina.*

11. Procédé de production de protéine d'intérêt selon les revendications 1 à 10, dans lequel la plante appartenant à la famille des *Brassicaceae* est *Brassica rapa* ou *Arabidopsis thaliana.*

12. Procédé de production de protéine d'intérêt selon les revendications 1 à 11, dans lequel la protéine d'intérêt est une protéine d'origine virale, notamment une protéine du virus de l'Hépatite B référencée sous le numéro d'accession SwissProt P03141.3, ou une protéine d'origine animale, notamment issue d'un mammifère, ledit mammifère étant choisi parmi les rongeurs, les félins, les canins et les primates, ou une protéine d'origine humaine, ou une protéine d'origine végétale, notamment les protéines glycosylées de plantes, la lectine ou la papaïne.

13. Procédé de production de protéine d'intérêt selon les revendications 1 à 12, dans lequel la protéine d'intérêt est choisie parmi les allergènes, les vaccins, les enzymes, les inhibiteurs d'enzyme, les anticorps, les fragments d'anticorps, les antigènes, les toxines, les peptides antimicrobiens, les hormones, les facteurs de croissance, les protéines du sang, les récepteurs, les protéines de signalisation, les composants protéiques d'étalon biomédicaux, les composants protéiques de milieu de culture cellulaire, les protéines de fusion ou marquées, les peptides ou protéines riches en cystéine.

14. Procédé de production de protéine d'intérêt selon les revendications 1 à 13, dans lequel la protéine d'intérêt est la lipase gastrique humaine.

15. Procédé de production d'émergence de racines latérales apparaissant sur des racines chevelues, en milieu liquide contenant au moins une auxine, comprenant les étapes de :
a) transformation d'une plante appartenant à la famille des *Brassicaceae* avec une souche *de Rhizobium* comprenant les gènes *rol,* afin d'obtenir les racines chevelues, et
b) induction d'émergences de racines latérales qui ne s'allongent pas sur les racines chevelues en présence d'au moins une auxine en milieu liquide.

## Patentansprüche

1. Verfahren zur Herstellung eines interessierenden Proteins aus Seitenwurzelaustritten, die an haarigen Wurzeln einer Pflanze entstehen, die zur Familie der *Brassicaceae* gehört, in flüssigem Medium, das mindestens ein Auxin enthält, umfassend die folgenden Schritte:
a) Transformieren einer Pflanze, die zur Familie der *Brassicaceae* gehört, mit einem *Rhizobium-Stamm,* der die *rol*-Gene umfasst, um die haarigen Wurzeln zu erhalten, und
b) Transformieren dieser Pflanze mit einem Vektor, der eine Expressionskassette enthält, die ein Gen umfasst, das für das interessierende Protein kodiert,
wobei diese Schritte in einem ersten Kulturmedium stattfinden, und
c) Induzieren von Seitenwurzelaustritten an den haarigen Wurzeln in Gegenwart von mindestens einem Auxin in flüssigem Medium, das ein zweites flüssiges Kulturmedium darstellt, und
d) Kultivieren der haarigen Wurzeln, die Seitenwurzelaustritte aufweisen, die sich nicht verlängern, und
e) spontanes Sezernieren des interessierenden Proteins in das Medium während der Kultivierung und
f) Gewinnen dieses interessierenden Proteins direkt aus dem zweiten flüssigen Kulturmedium und
g) gegebenenfalls Gewinnen dieses, im Gewebe akkumulierten interessierenden Proteins durch Zerkleinern der haarigen Wurzeln.

2. Verfahren zur Herstellung eines interessierenden Proteins aus Seitenwurzelaustritten, die an haarigen Wurzeln einer Pflanze entstehen, die zur Familie der *Brassicaceae* gehört, in flüssigem Medium, das mindestens ein Auxin enthält, nach Anspruch 1, umfassend die folgenden Schritte:
a) Transformieren einer Pflanze, die zur Familie der *Brassicaceae* gehört, mit einem *Rhizobium-Stamm,* der die *rol*-Gene umfasst, um die haarigen Wurzeln zu erhalten, und
b) Transformieren der Pflanze mit einem Vektor, der eine Expressionskassette enthält, die ein Gen umfasst, das für das interessierende Protein kodiert, und
c) Kultivieren der gemäß Schritt a) und b) erhaltenen haarigen Wurzeln in flüssigem Medium,
wobei diese Schritte in einem ersten Kulturmedium stattfinden, und
d) Induzieren von Seitenwurzelaustritten an den haarigen Wurzeln in Gegenwart von mindestens einem Auxin in flüssigem Medium, das ein zweites flüssiges Kulturmedium darstellt, und
e) Kultivieren der haarigen Wurzeln, die Seitenwurzelaustritte aufweisen, die sich nicht verlängern, in dem zweiten flüssigen Kulturmedium, und
f) spontanes Sezernieren des interessierenden Proteins in das zweite flüssige Kulturmedium während der Kultivierung und
g) Gewinnen des interessierenden Proteins direkt aus dem zweiten flüssigen Kulturmedium und
h) gegebenenfalls Gewinnen dieses, im Gewebe akkumulierten interessierenden Proteins durch Zerkleinern der haarigen Wurzeln.

3. Verfahren zur Herstellung eines interessierenden Proteins nach Anspruch 1 und 2, wobei der *Rhizobium-*Stamm ein Stamm der Spezies *Rhizobium rhizogenes* ist, wobei der Stamm von *R. rhizogenes* insbesondere ausgewählt ist aus den Stämmen ATCC 25818, ICPB TR7, LBA 9402, A4T, A4, LBA1334, ATCC 11325, ATCC 15834 und LMG 155, und vorzugsweise ausgewählt ist aus dem Stamm ATCC 25818 oder dem Stamm ICPB TR7.

4. Verfahren zur Herstellung eines interessierenden Proteins nach den Ansprüchen 1 bis 3 in einem flüssigen Kulturmedium, das mindestens ein Auxin enthält, aus Seitenwurzelaustritten, die sich nicht verlängern, die an haarigen Wurzeln einer Pflanze entstehen, die zur Familie der *Brassicaceae* gehört, umfassend die folgenden Schritte:
a) Transformieren einer Pflanze, die zur Familie der *Brassicaceae* gehört, mit einem *Rhizobium-Stamm,* der die *rol*-Gene und eine Expressionskassette umfasst, die ein Gen umfasst, das für das interessierende Protein kodiert, um die haarigen Wurzeln zu erhalten, und
b) Kultivieren der gemäß Schritt a) erhaltenen haarigen Wurzeln in flüssigem Medium,
wobei diese Schritte in einem ersten Kulturmedium stattfinden, und
c) Induzieren von Seitenwurzelaustritten an den haarigen Wurzeln in Gegenwart von mindestens einem Auxin in flüssigem Medium, das ein zweites flüssiges Kulturmedium darstellt, und
d) Kultivieren der Seitenwurzelaustritte, die sich nicht verlängern, die an diesen haarigen Wurzeln entstehen, in dem zweiten flüssigen Kulturmedium und
e) spontanes Sezernieren des interessierenden Proteins in das zweite flüssige Kulturmedium während der Kultivierung und
f) gegebenfalls Gewinnen dieses interessierenden exprimierten Proteins durch Gewebeextraktion und direkt aus dem zweiten flüssigen Kulturmedium.

5. Verfahren zur Herstellung eines interessierenden Proteins nach den Ansprüchen 1 bis 4, wobei der Schritt des Induzierens von Seitenwurzelaustritten an den haarigen Wurzeln in Gegenwart von mindestens einem Auxin in flüssigem Medium durch Zugabe von mindestens einem Auxin in das erste flüssige Kulturmedium der haarigen Wurzeln durchgeführt wird, um das zweite flüssige Kulturmedium zu ergeben.

6. Verfahren zur Herstellung eines interessierenden Proteins nach den Ansprüchen 1 bis 5, wobei der Schritt des Induzierens von Seitenwurzelaustritten an den haarigen Wurzeln in Gegenwart von mindestens einem Auxin in flüssigem Medium durch Ersetzen eines ersten Kulturmediums der haarigen Wurzeln durch ein zweites flüssiges Kulturmedium, das mindestens ein Auxin enthält, durchgeführt wird.

7. Verfahren zur Herstellung eines interessierenden Proteins nach den Ansprüchen 1 bis 6, wobei der Schritt zum Gewinnen dieses interessierenden exprimierten Proteins direkt aus dem zweiten Kulturmedium nach einer spontanen Sekretion während der Kultivierung und optional durch Zerkleinern des kultivierten Gewebes durchgeführt wird.

8. Verfahren zur Herstellung eines interessierenden Proteins nach den Ansprüchen 1 bis 7, wobei das Auxin ausgewählt ist aus: 2,4-Dichlorphenoxyessigsäure (2,4-D), 3-Indolessigsäure (IAA), Indol-3-Buttersäure (IBA), 1-Naphthalinessigsäure (NAA), 2,4,5-Trichlorphenoxyessigsäure (2,4,5-T), 2,3,5-Triiodessigsäure, 4-Chlorphenoxyessigsäure, 2-Naphthoxyessigsäure, 1-Naphthylessigsäure, 4-Amino-3,5,6-trichlorpicolinsäure, 3,6-Dichlor-2-methoxybenzoesäuren (Dicamba).

9. Verfahren zur Herstellung eines interessierenden Proteins nach den Ansprüchen 1 bis 8, wobei 2,4-D-Auxin in einer Konzentration von 0,01 bis 10 mg/l, insbesondere 0,2 bis 1 mg/l, insbesondere 0,5 mg/l, insbesondere 1 mg/l, verwendet wird.

10. Verfahren zur Herstellung eines interessierenden Proteins nach den Ansprüchen 1 bis 9, wobei die Pflanze, die zur Familie der *Brassicaceae* gehört, ausgewählt ist aus den Gattungen *Arabidopsis, Armoracia, Barbarea, Brassica, Crambe, Eruca, Raphanus, Wasabia* und *Camelina.*

11. Verfahren zur Herstellung eines interessierenden Proteins nach den Ansprüchen 1 bis 10, wobei die Pflanze, die zur Familie der *Brassicaceae* gehört, *Brassica rapa* oder *Arabidopsis thaliana* ist.

12. Verfahren zur Herstellung eines interessierenden Proteins nach den Ansprüchen 1 bis 11, wobei das interessierende Protein ein Protein viralen Ursprungs, insbesondere ein Protein des Hepatitis-B-Virus, das unter der SwissProt-Zugangsnummer P03141.3 gelistet ist, oder ein Protein tierischen Ursprungs, insbesondere von einem Säugetier, wobei das Säugetier ausgewählt ist aus Nagetieren, Katzenartigen, Hundeartigen und Primaten, oder ein Protein menschlichen Ursprungs, oder ein Protein pflanzlichen Ursprungs ist, insbesondere glykosylierte Pflanzenproteine, Lektin oder Papain.

13. Verfahren zur Herstellung eines interessierenden Proteins nach den Ansprüchen 1 bis 12, wobei das interessierende Protein ausgewählt ist aus Allergenen, Impfstoffen, Enzymen, Enzyminhibitoren, Antikörpern, Antikörperfragmenten, Antigenen, Toxinen, antimikrobiellen Peptiden, Hormonen, Wachstumsfaktoren, Blutproteinen, Rezeptoren, Signalproteinen, Proteinbestandteilen biomedizinischer Standards, Proteinbestandteilen von Zellkulturmedien, Fusionsproteinen oder markierten Proteinen, Cysteinreichen Peptiden oder Proteinen.

14. Verfahren zur Herstellung eines interessierenden Proteins nach den Ansprüchen 1 bis 13, wobei das interessierende Protein menschliche Magenlipase ist.

15. Verfahren zur Herstellung von Seitenwurzelaustritten, die an haarigen Wurzeln entstehen, in flüssigem Medium, das mindestens ein Auxin enthält, umfassend die folgenden Schritte:
a) Transformieren einer Pflanze, die zur Familie der *Brassicaceae* gehört, mit einem *Rhizobium-Stamm,* der die *rol*-Gene umfasst, um die haarigen Wurzeln zu erhalten, und
b) Induzieren von Seitenwurzelaustritten, die sich nicht verlängern, an den haarigen Wurzeln in Gegenwart von mindestens einem Auxin in flüssigem Medium.

## Claims

1. Method for producing protein of interest from lateral root emergences appearing on hairy roots of a plant belonging to the family of the *Brassicaceae,* in liquid medium containing at least one auxin, comprising the steps of:
a) transforming a plant belonging to the family of the *Brassicaceae* with a strain of *Rhizobium* comprising the *rol* genes, in order to obtain the hairy roots, and
b) transforming said plant with a vector containing an expression cassette comprising a gene encoding said protein of interest,
the aforesaid steps taking place in a first culture medium, and
c) inducing lateral root emergences on the hairy roots in the presence of at least one auxin in liquid medium constituting a second liquid culture medium, and
d) culture of the hairy roots having lateral root emergences that do not grow longer, and
e) the spontaneous secretion in the medium of the protein of interest during the culture, and
f) the recovery of the aforesaid protein of interest directly from the second liquid culture medium and
g) optionally, the recovery of the aforesaid protein of interest that has accumulated in the tissues by grinding the hairy roots.

2. Method for producing protein of interest from lateral root emergences appearing on hairy roots of a plant belonging to the family of the *Brassicaceae,* in liquid medium containing at least one auxin, according to claim 1, comprising the steps of:
a) transforming a plant belonging to the family of the *Brassicaceae* with a strain of *Rhizobium* comprising the *rol* genes, in order to obtain the hairy roots, and
b) transforming said plant with a vector containing an expression cassette comprising a gene encoding said protein of interest, and
c) culture in liquid medium of the hairy roots obtained according to steps a) and b),
the aforesaid steps taking place in a first culture medium, and
d) inducing lateral root emergences on the hairy roots in the presence of at least one auxin in liquid medium constituting a second liquid culture medium, and
e) culture in the second liquid culture medium, of the hairy roots having lateral root emergences that do not grow longer, and
f) the spontaneous secretion in the second liquid culture medium, of the protein of interest during the culture, and
g) the recovery of the protein of interest directly from the second liquid culture medium, and
h) optionally, the recovery of the aforesaid protein of interest that has accumulated in the tissues by grinding the hairy roots.

3. Method for producing protein of interest according to claims 1 and 2, in which the strain of Rhizobium is a strain of the species *Rhizobium rhizogenes,* in particular in which said strain of *R. rhizogenes* is selected from the strains ATCC 25818, ICPB TR7, LBA 9402, A4T, A4, LBA1334, ATCC 11325, ATCC 15834 and LMG 155 and is preferentially selected from the strain ATCC 25818 or the strain ICPB TR7.

4. Method for producing protein of interest according to claims 1 to 3, in a liquid culture medium containing at least one auxin, from lateral root emergences that do not grow longer appearing on hairy roots of a plant belonging to the family of the *Brassicaceae,* comprising the steps of:
a) transforming a plant belonging to the family of the *Brassicaceae* with a strain of *Rhizobium* comprising the *rol* genes and an expression cassette comprising a gene encoding said protein of interest in order to obtain the hairy roots, and
b) culture in liquid medium of the hairy roots obtained according to step a),
the aforesaid steps taking place in a first culture medium, and
c) inducing lateral root emergences on the hairy roots in the presence of at least one auxin in liquid medium, constituting a second culture medium, and
d) culture in the second liquid culture medium, of the lateral root emergences that do not grow longer appearing on the aforesaid hairy roots, and
e) the spontaneous secretion in the second liquid culture medium, of the protein of interest during the culture, and
f) optionally, the recovery of the aforesaid expressed protein of interest by extraction from the tissues and directly from the second liquid culture medium.

5. Method for producing protein of interest according to claims 1 to 4, in which the step of inducing lateral root emergences on the hairy roots in the presence of at least one auxin in liquid medium is carried out by the addition of at least one auxin to the first liquid culture medium of the hairy roots, in order to produce the second liquid culture medium.

6. Method for producing protein of interest according to claims 1 to 5, in which the step of inducing lateral root emergences on the hairy roots in the presence of at least one auxin in liquid medium is carried out by replacing a first liquid culture medium of the hairy roots with a second liquid culture medium containing at least one auxin.

7. Method for producing protein of interest according to claims 1 to 6, in which the step of recovery of the aforesaid expressed protein of interest is carried out directly from the second culture medium after a spontaneous secretion during the culture and optionally by grinding of the cultured tissues.

8. Method for producing protein of interest according to claims 1 to 7, in which the auxin is selected from: 2,4-dichlorophenoxyacetic acid (2,4-D), 3-indoleacetic acid (IAA), indole-3-butyric acid (IBA), 1-naphthaleneacetic acid (NAA), 2,4,5-trichlorophenoxyacetic acid (2,4,5-T), 2,3,5-triiodoacetic acid, 4-chlorophenoxyacetic acid, 2-naphthoxyacetic acid, 1-naphthylacetic acid, 4-amino-3,5,6-trichloropicolinic acid, 3,6-dichloro-2-methoxybenzoic acid (Dicamba).

9. Method for producing protein of interest according to claims 1 to 8, in which the 2,4-D auxin is used at a concentration from 0.01 to 10 mg/l, in particular from 0.2 to 1 mg/l, in particular 0.5 mg/l, in particular 1 mg/l.

10. Method for producing protein of interest according to claims 1 to 9, in which the plant belonging to the family of the *Brassicaceae* is selected from the genera *Arabidopsis, Armoracia, Barbarea, Brassica, Crambe, Eruca, Raphanus, Wasabia* and *Camelina.*

11. Method for producing protein of interest according to claims 1 to 10, in which the plant belonging to the family of the *Brassicaceae* is *Brassica rapa* or *Arabidopsis thaliana.*

12. Method for producing protein of interest according to claims 1 to 11, in which the protein of interest is a protein of viral origin, in particular a protein of the hepatitis B virus referenced by Swiss-Prot accession number P03141.3, or a protein of animal origin, in particular originating from a mammal, said mammal being selected from the rodents, felines, canines and primates, or a protein of human origin, or a protein of plant origin, in particular the glycosylated plant proteins, lectin or papain

13. Method for producing protein of interest according to claims 1 to 12, in which the protein of interest is selected from the allergens, vaccines, enzymes, enzyme inhibitors, antibodies, antibody fragments, antigens, toxins, antimicrobial peptides, hormones, growth factors, blood proteins, receptors, signalling proteins, protein components of biomedical standards, protein components of cell culture medium, fusion or labelled proteins, cysteinerich peptides or proteins.

14. Method for producing protein of interest according to claims 1 to 13, in which the plant of interest is human gastric lipase.

15. Method for producing lateral root emergences appearing on hairy roots, in liquid medium containing at least one auxin, comprising the steps of:
a) transforming a plant belonging to the family of the *Brassicaceae* with a strain of *Rhizobium* comprising the *rol* genes, in order to obtain the hairy roots, and
b) inducing lateral root emergences that do not grow longer on the hairy roots in the presence of at least one auxin in liquid medium.
